# EUROPEAN PATENT APPLICATION

(11) **EP 4 464 713 A1**
(43) Date of publication of application: **20.11.2024**
(21) Application number: 23461592.0
(22) Date of filing: 18.05.2023
(51) Int. Cl.: C07H 1/00, C07H 21/02, C09B 23/06, C09B 23/08, C12N 15/11, A61K 48/00, A61K 31/712

(54) **CIRCULAR RNA ANALOGS, PREPARATION AND APPLICATION THEREOF**

(71) Applicant: Siec Badawcza Lukasiewicz - PORT Polski Osrodek Rozwoju Technologii, 54-066 Wroclaw (PL)
(72) Inventor: Mamot, Adam, 01-930 Warszawa (PL); Kowalska, Joanna, 02-109 Warszawa (PL); Jemielity, Jacek, 02-353 Warszawa (PL); Wasinska-Kalwa, Malgorzata, 03-808 Warszawa (PL); Nowis, Dominika, 02-392 Warszawa (PL); Czubak, Karol, 26-115 Skar ysko Ko cielne (PL); Golab, Jakub, 02-392 Warszawa (PL)
(74) Representative: Witek, Rafal

(57) **Abstract**

The presented invention relates to a novel class of nucleic acids, which have circular topology and contain at least one unnatural internucleotide linkage. The main objects of the disclosed invention are analogs of circular RNA (circRNA) that are obtained by the synthesis of 5'-modified precursors (pre-circRNAs) and their subsequent circularization via a chemical reaction.

## Description

### Summary of the invention

The presented invention relates to a novel class of nucleic acids, which have circular topology and contain at least one unnatural internucleotide linkage. The main objects of the disclosed invention are analogs of circular RNA (circRNA) that are obtained by the synthesis of 5'-modified precursors (pre-circRNAs) and their subsequent circularization via a chemical reaction. The circularization occurs due to the presence of two chemically reactive functional groups, one at RNA 5' end and another at the 3' end. The functional group at the 5' end is incorporated co-transcriptionally, whereas the reactive group at the 3' end is incorporated post-transcriptionally. The invention encompasses RNAs of different lengths (from 2 nt to 20000 nt), including messenger RNAs (RNAs encoding a protein). The circRNAs encoding a protein according to this invention undergo translation with an efficiency higher than chemically unmodified circRNAs of the same sequence. One of the embodiments of the invention discloses circRNA analogs that are obtained from pre-circRNAs containing a functionalized 7-methylguanosine cap analog at their 5' end. Such circRNAs containing caps are more efficiently translated than circRNAs of the same sequence without the cap. Methods of preparation of circRNA analogs, methods of preparation of corresponding pre-circRNAs, methods of preparation of a protein or peptide, and application of the disclosed circRNA analogs in the context of gene therapies and vaccinations are also disclosed.

### Field of the invention

The presented invention relates to RNA molecules, methods of producing circular RNA molecules, methods of producing a protein or peptide in vitro, in cells, or living organisms, and methods of treating or preventing a disease.

### Background of the invention

Despite its inherent fragility, RNA is one of the most potent substances in the context of therapeutic gene delivery.¹ The potency of therapeutic messenger RNA (mRNA) stems from both advanced methods of delivery and chemical modification of the mRNA itself. Both in serum and in the cytosol, mRNA is primarily targeted by exonucleases (e.g. decapping complexes, deadenylases, exosomes).² Circularization is one of the most protective modifications of mRNA, as it prevents exonucleolytic digestion.³

Currently known methods of RNA circularization are based on chemical reactions^{4, 5}, enzymatic reactions⁶⁻⁹, and reactions catalyzed by catalytic sequences embedded in circRNA precursors¹⁰⁻¹³ (pre-circRNA).

The reported methods of circularization that are based on chemical reactions rely on the use of short RNA sequences, chemically synthesised via phosphoramidite method, such as 3' phosphorylated RNA or 3'-amino RNA, which undergo condensation induced by cyanogen bromide (BrCN) or 1-ethyl-3-(3-dimethylaminopropyl)carbodiimide (EDC).^{4, 5} The phosphoramidite solid phase synthesis of nucleic acids is a robust method of production of modified and unmodified oligonucleotides, however it is not practical for production of long (> 100 nt) RNA sequences. In consequence, circRNA obtained with these methods encode relatively short sequences and have limited use in the context of RNAs encoding a protein or peptide, which are usually significantly longer.

Moreover, RNA circularization can be caried out with ligases, especially T4 RNA ligase I and T4 RNA ligase II.^{6, 7, 14} Both enzymes require 5' monophosphorylated precursors, that are first adenylated and subsequently ligated with the 3' OH of the ribose at the 3' end of a different or the same RNA strand.¹⁵ Both enzymes are capable of intramolecular ligation (circularization) of RNA (if reaction occurs with the 3' end of the same RNA strand); however, they have been found strongly dependent on target RNA length and sequence.^{11, 12, 14, 16} This is detrimental, as it difficult to predict or improve performance of such enzymes.

The most recognised methods of RNA circularization involve catalytic nucleic acids.^{8-12, 17} Wesselhoeft *et al.* have circularized RNA *in vitro* by using the permuted intron-exon (PIE) system and optimized self-splicing intronic sequences.^{11, 12} The PIE system consists of fused partial exons flanked by half-intron sequences.^{10, 17} During the self-splicing reaction, two transesterification reactions occur. First, 3' hydroxyl group of a guanosine nucleotide engages 5' splice site. Second, the freed hydroxyl group at the end of the intermediate engages the 3' splice site, which results in circularization.

Litke *et al.* have shown the Twister-optimized RNA durable overexpression (Tornado) system for RNA circularization.⁸ Tornado-expressed transcripts contain an RNA of interest flanked by Twister ribozymes that spontaneously undergo autocatalytic cleavage. This leaves 5'-hydroxyl and 2',3'-cyclic phosphate termini that are ligated by the RNA ligase RtcB.

The so-far-reported methods based on catalytic nucleic acid activity require sequence design and development, therefore cannot be used on pre-circRNA of any given sequence. Furthermore, pre-circRNA's ability to undergo circularization can be hindered by the presence of chemical modifications, such as natural and synthetic nucleosides and nucleobases.¹²

One of the biggest flaws of circRNA in the context of gene delivery and gene therapies is low protein expression. The main pipelines of eukaryotic translational machinery are based on cap-dependent mechanisms.^{18, 19} None of the discovered or synthesised circular RNA (circRNA) contain elements such as 7-methylguanosine cap, or any kind of 5' nucleoside analog.^{20, 21} Due to circular and "cap-less" structure, translation of currently known circRNA occurs in an IRES (internal ribosome entry site)-dependent manner.^{5, 7, 9, 11, 12, 19, 22, 23} In eukaryotic cells, cap-dependent translation is much more effective IRES-dependent.¹⁹ On the other hand, the stability of circRNA is higher, in consequence, circRNAs exhibit significantly longer half-lives and the protein expression from these mRNAs lasts longer.^{12, 14}

WO/2021/113777 discloses circular RNAs and transfer vehicles, along with related compositions and methods of treatment. Pharmaceutical compositions comprising such circular RNAs as well as precursor RNAs and materials useful in producing the precursor or circular RNAs have been also disclosed in cited document.

WO/2019/236673 describes a vector for making circular RNA allowing production of a circular RNA that is translatable or biologically active inside eukaryotic cells.

EP4008336 discloses a recombinant nucleic acid molecule of the transcriptional circular RNA, recombinant expression vector, pre-circularized RNA, circular RNA, recombinant host cell, pharmaceutical composition and protein preparing method. The transcription product of the recombinant nucleic acid molecule is a circular RNA which containing specific IRES element.

US20150079630 discloses cyclic RNA and method for production thereof comprising using of T4 DNA ligase.

None of the previously reported methods are suitable for incorporation of 7-methylguanosine cap structure within protein-coding circRNA.

### Summary

The present disclosure provides novel analogs of circRNAs that can be prepared from pre-circRNAs via a chemical reaction (chemical circularization of RNA), regardless of circRNA sequence, length or size. The proposed chemical circularization of RNA is compatible with the presence of other chemical modifications of the RNA body, such as natural and synthetic nucleosides and nucleobases, or fluorescent tags. Moreover, due to chemical nature of the circularization process, it enables incorporation of 7-methylguanosine cap within the structure of circRNA. Unexpectedly, the presence of the cap structure within a cricRNA analog molecule increases its translational activity, in comparison to chemically unmodified circRNAs of the same sequence. Therefore, present disclosure gives unprecedented access to circRNAs that undergo translation via cap-dependent mechanism.

The present disclosure provides a circRNA analog prepared by in vitro transcription reaction and a subsequent chemical circularization reaction wherein said circularization is obtained in the absence of protein enzymes or catalytic nucleic acid sequences.

The present disclosure provides a circRNA analog, that contains either a 7-methylguanosine cap analog structure or both a 7-methylguanosine cap analog structure and an internal ribosomal entry site sequence.

The present disclosure provides a circRNA analog according to formula 1 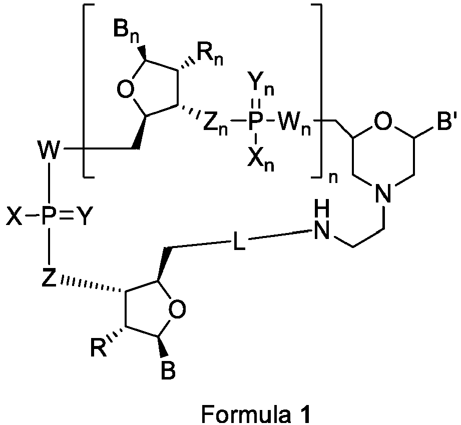 wherein:
n = 1-20 000;
X and each of X₁ through Xₙ is independently OH, BH₃, NH₂, or SH;
Y and each of Y₁ through Yₙ is independently O or S;
Z and each of Z₁ through Zₙ is independently O, S or NH;
Wand each of W₁ through Wₙ is independently O, S or NH;
R and each of R₁ through Rₙ is independently H, OH, alkyl, O-alkyl or halogen;
B, B' and each of B₁ through Bₙ is independently a natural, modified or unnatural nucleoside base;
L is a linear or branched linker.

In some embodiments, the linker L comprises of a natural, modified or unnatural 7-methylguanosine cap structure.

In some embodiments, the linker L comprises of a detectable label.

The present disclosure provides a circRNA analog according to formula 2 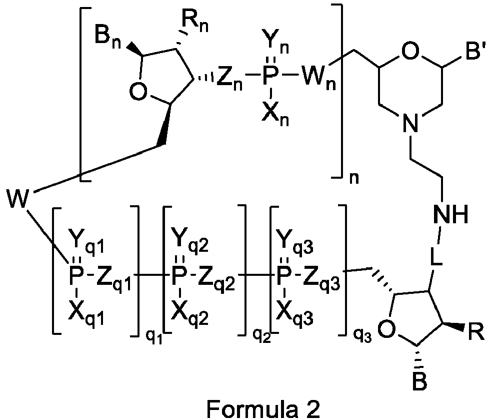 wherein:
n = 1-20 000;
each of q₁ through q₃ is independently 0 or 1;
X_{q1}, X_{q2}, X_{q3} and each of X₁ through Xₙ is independently OH, BH₃, NH₂, or SH;
Y_{q1}, Y_{q2}, Y_{q3} and each of Y₁ through Yₙ is independently O or S;
Z_{q1}, Z_{q2}, and Z_{q3} is independently O, S, NH, CH₂, C(halogen)₂ or CH(halogen);
each of Z₁ through Zₙ is independently O, S, or NH;
Wand each of W₁ through Wₙ is independently O, S or NH;
R and each of R₁ through Rₙ is independently H, OH, alkyl, O-alkyl or halogen;
B, B' and each of B₁ through Bₙ is independently a natural, modified or unnatural nucleoside base;
L is a linear or branched linker.

The present disclosure provides a circRNA analog according to formula 3 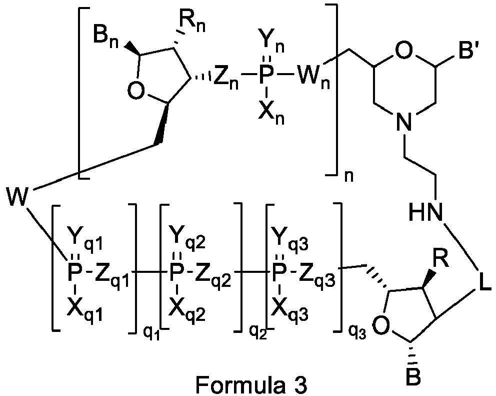 wherein:
n = 1-20 000;
each of q₁ through q₃ is independently 0 or 1;
X_{q1}, X_{q2}, X_{q3} and each of X₁ through Xₙ is independently OH, BH₃, NH₂, or SH;
Y_{q1}, Y_{q2}, Y_{q3} and each of Y₁ through Yₙ is independently O or S;
Z_{q1}, Z_{q2}, and Z_{q3} is independently O, S, NH, CH₂, C(halogen)₂ or CH(halogen);
each of Z₁ through Zₙ is independently O, S, or NH;
Wand each of W₁ through Wₙ is independently O, S or NH;
R and each of R₁ through Rₙ is independently H, OH, alkyl, O-alkyl or halogen;
B, B' and each of B₁ through Bₙ is independently a natural, modified or unnatural nucleoside base;
L is a linear or branched linker.

The present disclosure provides a circRNA analog according to formula 4 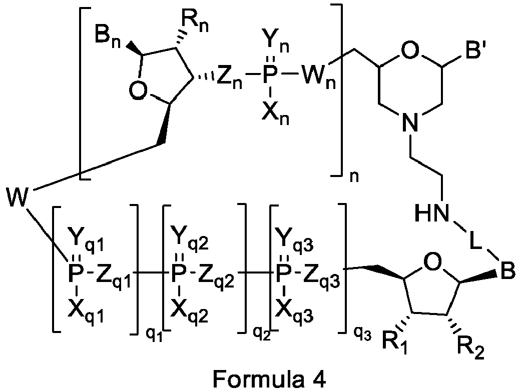 wherein:
n = 1-20 000;
each of q₁ through q₃ is independently 0 or 1;
X_{q1}, X_{q2}, X_{q3} and each of X₁ through Xₙ is independently OH, BH₃, NH₂, or SH;
Y_{q1}, Y_{q2}, Y_{q3} and each of Y₁ through Yₙ is independently O or S;
Z_{q1}, Z_{q2}, and Z_{q3} is independently O, S, NH, CH₂, C(halogen)₂ or CH(halogen);
each of Z₁ through Zₙ is independently O, S, or NH;
Wand each of W₁ through Wₙ is independently O, S or NH;
R and each of R₁ through Rₙ is independently H, OH, alkyl, O-alkyl or halogen;
B' and each of B₁ through Bₙ is independently a natural, modified or unnatural nucleoside base;
L is a linear or branched linker;
B is a modified or unnatural nucleoside base connected with the linker moiety L.

The present disclosure provides a circRNA analog according to formula 5 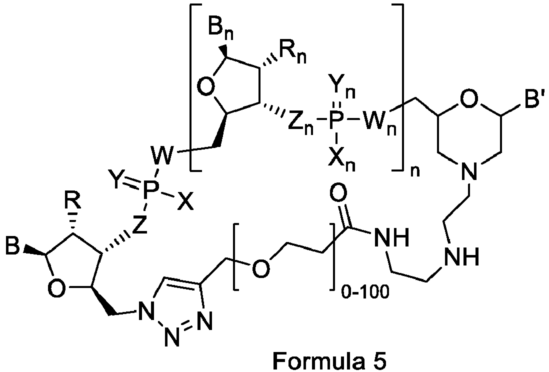 wherein:
n = 1-20 000
X and each of X₁ through Xₙ is independently OH, BH₃, NH₂, or SH;
Y and each of Y₁ through Yₙ is independently O or S;
Z and each of Z₁ through Zₙ is independently O, S or NH;
Wand each of W₁ through Wₙ is independently O, S or NH;
R and each of R₁ through Rₙ is independently H, OH, alkyl, O-alkyl or halogen;
B, B' and each of B₁ through Bₙ is independently a natural, modified or unnatural nucleoside base;

The present disclosure provides a circRNA analog according to formula 6 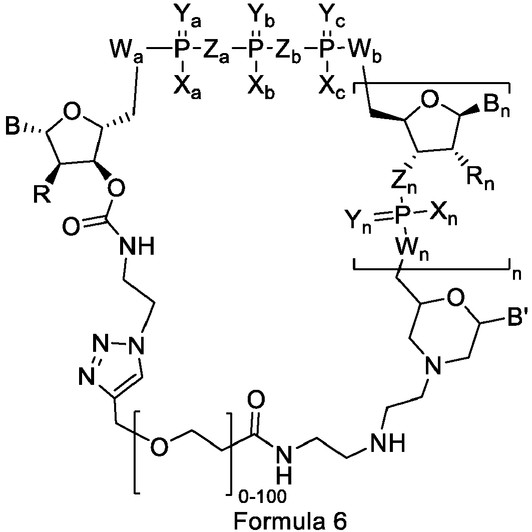 wherein:
n = 1-20 000;
Xₐ, X_{b}, X_{c} and each of X₁ through Xₙ is independently OH, BH₃, NH₂, or SH;
Yₐ, Y_{b}, Y_{c} and each of Y₁ through Yₙ is independently O or S;
each of Z₁ through Zₙ is independently O, S, or NH;
Xₐ and X_{b}, is independently O, S, NH, CH₂, C(halogen)₂ or CH(halogen);
Wₐ, W_{b} and each of W₁ through Wₙ is independently O, S or NH;
R each of R₁ through Rₙ is independently H, OH, alkyl, O-alkyl or halogen;
B, B' and each of B₁ through Bₙ is independently a natural, modified or unnatural nucleoside base;

The present disclosure provides a circRNA analog according to formula 6 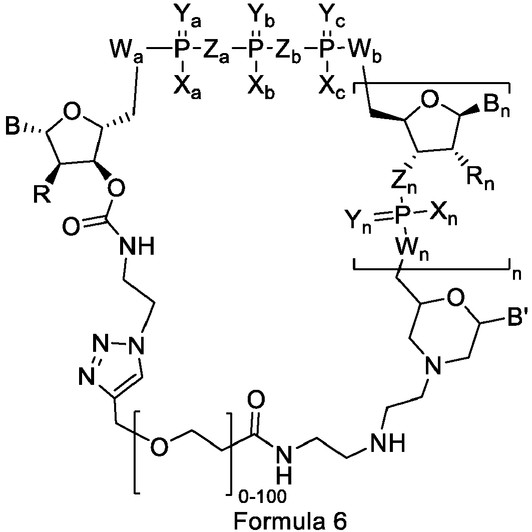 wherein:
n = 1-20 000;
Xₐ, X_{b}, X_{c} and each of X₁ through Xₙ is independently OH, BH₃, NH₂, or SH;
Yₐ, Y_{b}, Y_{c} and each of Y₁ through Yₙ is independently O or S;
each of Z₁ through Zₙ is independently O, S, or NH;
Xₐ and X_{b}, is independently O, S, NH, CH₂, C(halogen)₂ or CH(halogen);
Wₐ, W_{b} and each of W₁ through Wₙ is independently O, S or NH;
R each of R₁ through Rₙ is independently H, OH, alkyl, O-alkyl or halogen;
B, B' and each of B₁ through Bₙ is independently a natural, modified or unnatural nucleoside base;

The present disclosure provides a method of synthesis of circRNA analogs described above, characterized by that a circRNA analog precursor (formula 7) 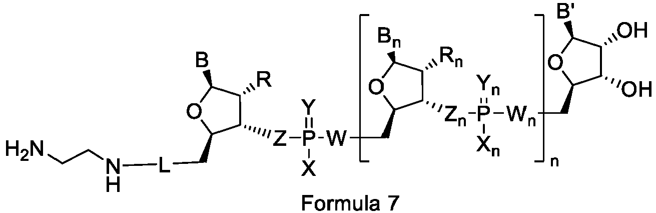 wherein:
n = 1-20 000;
X and each of X₁ through Xₙ is independently OH, BH₃, NH₂, or SH;
Y and each of Y₁ through Yₙ is independently O or S;
Z and each of Z₁ through Zₙ is independently O, S or NH;
Wand each of W₁ through Wₙ is independently O, S or NH;
R and each of R₁ through Rₙ is independently H, OH, alkyl, O-alkyl or halogen;
B, B' and each of B₁ through Bₙ is independently a natural, modified or unnatural nucleoside base;
L is a linear or branched linker;
is first oxidized during step (i) of the method, yielding a dialdehyde derivative (formula 8), 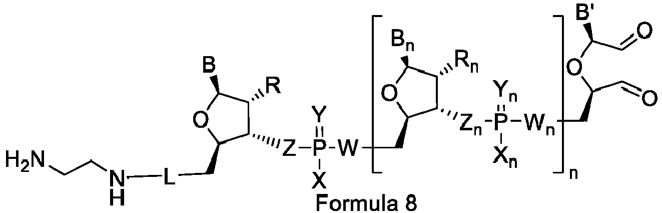 wherein:
   n = 1-20 000;
   X and each of X₁ through Xₙ is independently OH, BH₃, NH₂, or SH;
   Y and each of Y₁ through Yₙ is independently O or S;
   Z and each of Z₁ through Zₙ is independently O, S or NH;
   Wand each of W₁ through Wₙ is independently O, S or NH;
   R and each of R₁ through Rₙ is independently H, OH, alkyl, O-alkyl or halogen;
   B, B' and each of B₁ through Bₙ is independently a natural, modified or unnatural nucleoside base;
   L is a linear or branched linker;
   which is next incubated in reductive environment during step (ii) of the method, yielding the desired circRNA analog.

In some embodiments, step (i) and step (ii) are carried out in one reactor. In some embodiments, step (i) of the method is carried out in the presence of NaIO₄, preferably at a concentration below 10 mM, at a temperature below 60°C. In some embodiments, step (ii) of the method is carried out in buffered solution, preferably at pH 5-8, in presence of NaBH₃CN below 100 mM. In some embodiments, step (i) or step (ii) of the method are carried out in presence of a natural, modified or unnatural nucleic acid oligomer that is complementary to a RNA precursor sequence. In some embodiments, the circRNA analog is isolated from the reaction mixture by a known method of RNA isolation, such as precipitation in alcohol, size exclusion chromatography, gel electrophoresis, or high-performance liquid chromatography (HPLC).

The present disclosure provides a use of a circRNA analog described above in protein production in an *in vitro* setup.

The present disclosure provides a circRNA analog described above for use in therapeutic or diagnostic method, in particular for protein production in an *in vivo* setup.

The present disclosure provides a pharmaceutical composition comprising of a circRNA analog described above.

### Definitions

Unless defined otherwise, all terms used herein have the same meaning as are commonly understood by one of skill in the art to which this invention belongs.

The terms "nucleic acid" and "nucleic acid polymer" refer to a polymeric substance composed of ribo or deoxyribo derivatives and of naturally occurring or synthetic nucleoside monomers, including noncanonical linkages and any degree of polymerization. These phrases include but are not limited to natural or synthetic RNA and DNA, as well as nucleic acids composed of phosphorothioate, boranophosphate, selenophosphate, and phosphonate moieties, morpholino nucleic acids (MNA), locked nucleic acid (LNA), peptide nucleic acid (PNA), and threose nucleic acid (TNA).

The terms "modification", "modifying group", and "modified" in the context of nucleic acids refer to any chemical moiety that may be attached to a nucleic acid molecule. This includes but is not limited to attachment of the modifying group to the sugar, nucleobase, internucleotide phosphate, or 5'-5'-oligophosphate of a nucleic acid molecule.

The term "internucleotide linkage" refers to a covalent or non-covalent bond or bonds that connect two monomers of a nucleic acid polymer.

The terms "nucleoside base", "nucleobase" and "nitrogenous base" refer to a part of nucleic acid molecules. The referred nucleobases may be involved in base pairing, base stacking, and other inter- and intramolecular interactions. The referred nucleobases include but are not limited to natural, modified, unnatural or synthetic purine (such as adenine, guanine, hypoxanthine, xanthine , N7-methylguanosine, N6-methyladenosine, N2-methyladenosine) and pyrimidine (such as cytosine, thymidine, uracil, dihydrouracil, 5-methylcytosine, 5-hydroxymethylcytosine, pseudouridine, methylpseudouridine, nicotinamide) nucleobases, as well as 2-aminopurine, 2,6-diaminopurine, flavin, C8-substituted adenine, C8-substituted guanine, C7-substituted 7-deazaadenine, C7-substituted 7-deazaguanine, C5-substituted uridine, C5-substituted cytosine, di- and tricyclic nucleobases.

The terms "nucleic acid sequence", "nucleotide sequence", "nucleoside sequence", "nucleobase sequence" and "sequence" refer to the order and composition of a series of nucleobases within a nucleic acid molecule.

The terms "nucleobase pair" and "base pair" refer to a fundamental unit of double-stranded nucleic acids consisting of two nucleobases bound to each other by hydrogen bonds. The term "base pairing" refers to specific hydrogen bonding between nucleobases, that can be canonical (Watson-Crick base paring) or non-canonical (e.g. wobble base paring, Hoogsten base paring).

The terms "hybridisation" and "hybridize" refer to interaction of two or more nucleic acid strands. It is characterized by that, that under appropriate conditions distinct nucleic acid strands maintain a sequence-dependent structure (e.g. a double helix, a triple helix, an I-motif, a G-quadruplex).

The term "complementary" refers to relation between sequences of two or more strands of a nucleic acid or nucleic acids. The complementary sequences or strands are characterised by that they undergo hybridization and form a sequence-dependent structure. The complementarity can be full or partial. The terms "linear" and "circular" in context of nucleic acids refer to the topology of a nucleic acid molecule. Linear nucleic acids are linear polymers with no linkage between 5' and 3' ends. Circular nucleic acids are polymers with natural or modified linkage between 5' and 3' ends.

The terms "circularization" and "cyclization" refer to a process of transformation of a linear nucleic acid polymer to a circular nucleic acid polymer.

The terms "circRNA precursor" and "pre-circRNA" refer to a linear nucleic acid polymer to that can undergo circularization leading to a circular nucleic acid polymer.

The term "linker" in context of circular nucleic acids refers to any natural or synthetic combination of chemical moieties that covalently join 5' and 3' ends of the polymer. The term "linker" includes, but is not limited to any linear or branched combination of alkyl, alkenyl, alkynyl, ether, ester, amide, amine, carbamate, triazol, phosphoester, amidophosphate, or phosphonate moieties. In particular it can be selected among of following structures: 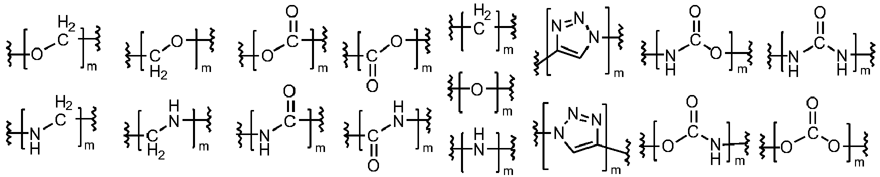 wherein m is a natural number from 1 to 100.

The term "cap analog" refers to any compound that contains natural or modified 5'-5'-oligophosphate linking a natural or modified nucleosides, nucleotides, or nucleic acids. The term "Cap analog" refers to canonical, as well as non-canonical cap structure and includes, but is not limited to Cap0, Cap1, Cap2, TMG-cap, NAD-cap, and FAD-cap, as well as cap structures modified within oligophosphate bridge, ribose, or nucleobases. In particular, it can be selected among of structures described in any document cited in subject description, esspecially: WO/2009/149253, WO/2008/157688, WO/2011/015347, WO/2022/191725, WO/2018/015845, WO/2014/093627, WO/2021/162566, WO/2021/162567 and WO/2017/130151.

The terms "internal ribosomal entry site" and "IRES" refer to RNA sequences that are characterised by their ability facilitate cap-independent initiation of translation by interaction or binding cellular proteins involved in formation of ribosomes and translation. The term "IRES sequence" includes, but is not limited to Encephalomyocarditis virus (EMCV) IRES, Coxsackievirus B3 (CVB3) IRES, respiratory syncytial virus (RSV) m⁶A methylation sites, viral IRES sequences, human IRES sequences, m⁶A methylation sites, sequences containing m⁶A modification, eIF4G aptamers, and artificial IRES sequences.^{11, 12, 22, 24}

The terms "transcription" or "transcription reaction" refer to methods for enzymatic synthesis of RNA that is complementary to a DNA template. Transcription can occur in cells, in cellular systems or in artificial setups (as in *in vitro* transcription reaction). Transcription is carried out in presence of a DNA-dependent RNA polymerase, ribonucleoside triphosphates (NTPs), and a DNA template. During the reaction, the sequence of the DNA template is copied and amplified in form of RNA molecules. Optionally, the transcription can be carried out in the presence of additional reagents, such as transcription initiators, transcription terminators, or modified nucleoside triphosphates.

The terms "label" and "detectable label" refer to any compound, particle, or any combination of compounds and/or particles, that may be directly or indirectly detected. The label can be associated with a target molecule by covalent and/or non-covalent interactions. The label can be a radioisotope, NMR label, spin label, a chromophore, a fluorophore, a hapten. The preferred label includes but is not limited to ²H, ¹³C, ¹⁵C, ³²P, ¹⁹F, cyanines, fluoresceines, rhodamines, coumarines, perylenes, naphthalimides, nitro dyes, azo dyes, BODIPY, biotin, desthiobiotin, folic acid and N-acetylgalactosamine.

The terms "periodate oxidation", "cis-diol oxidation" and "ribose oxidation" refer to a process of cleavage of carbon-carbon bonds of vicinal cis-diols present in the structure of a natural or synthetic nucleic acids leading to a dialdehyde derivative of a nucleic acid.

The terms "reductive amination" and "amination" in the context of nucleic acid modification or circularization refer to amination reaction involving a dialdehyde derivative of a nucleic acid.

The term "reductive environment" refers to conditions under which chemical reduction reactions occur. The referred reductive environment includes but is not limited to the presence of sodium cyanoborohydride, sodium borohydride, pyridine borane, or sodium ascorbate.

The terms "halo" and "halogen" refer to all halogens, such as chloro, fluoro, bromo, and iodo groups.

The term "alkyl" refers to a single bond chain of hydrocarbons ranging from 1 to 20 carbon atoms and includes but is not limited to methyl, ethyl, propyl, isopropyl, n-butyl, sec-butyl, isobutyl, tert-butyl, pentyl, isoamyl, hexyl, octyl, and dodecanyl groups.

The term "O-alkyl" denotes the group -OR , where R is an alkyl, and includes but is not limited to O-methoxy, O-ethoxy, O-propoxy, O-isopropoxy, O-butoxy, O-isobutoxy, and O-pentoxy groups.

The terms "ethylenediamine motif', "EDA motif', "EDA group", and "EDA" refer to a chemical motif of R-CH₂-NH-(CH₂)₂-NH₂, where R is any substituent. Structure of the EDA motif may as well be recognised in an ionic form or in a form of a salt.

The term "reactive functional group" refers to any functional group that is incorporated at the RNA 5' or 3' end that can react with another (same or different) reactive group in a selective manner in presence of other (natural or unnatural) chemical moieties contained within structure of the circRNA precursor. The representative pairs of 5' and 3' reactive groups which are reactive towards each other, include but are not limited to EDA and dialdehyde, hydrazide and dialdehyde, azide and alkyne, azide and cyclooctyne derivative, amino and carboxylic group, amino and fluorosulfonylgroup, azide and phosphine, etc.

The terms "nucleic acids alcohol precipitation", "nucleic acids precipitation", and "alcohol precipitation" refer to the process of nucleic acid isolation. It is a technique to de-salt and concentrate nucleic acids from aqueous solutions. Addition of polar organic solvents (e. g. ethanol, isopropanol, acetone) and salts (e. g. sodium acetate, ammonium acetate, lithium chloride, sodium perchlorate, lithium perchlorate) to nucleic acid solution causes the precipitation of nucleic acids.

The terms "polyacrylamide gel electrophoresis" and "PAGE" refer to a technique used for separation of biological macromolecules according to their electrophoretic mobility. Electrophoretic mobility is a function of the mass, conformation, topology, and charge of the molecule. When PAGE is performed in denaturing conditions, it provides information on the composition of the nucleic acid species in the sample.

The terms "reversed-phase high pressure chromatography", "RP-HPLC, and "HPLC" refer to a technique for separation, identification, quantification, and isolation of components of a sample mixture. It relies on pumps constituting a flow of pressurized liquid mobile phase (a mixture of solvents and solutions) through a solid matrix of the stationary phase. Components of the sample interact differently with mobile and stationary phases and are in equilibrium between adsorbed and solubilized states. This is facilitated by a combination of hydrophobic, dipole-dipole and ionic interactions. As chromatography progresses, the analytes are resolved based on their elution velocity, can be detected by their molecular properties, and collected into separate fractions (peaks). The elution velocity of individual components of the sample is parameterized by the retention time of the fraction and the peak shape. The active component of the stationary phase is a monolith or particle solid resin of organic and/or inorganic composition, such as silica, poli(styren-co-divinylbenzen), or other polymers. The mobile phase is an aqueous or organic solution or a mixture of aqueous and organic solutions. The chromatography may be performed in ion-pair mode, in elevated temperatures, and for analytical as well as preparative purposes.

The terms "translation *in vitro",* "protein synthesis *in vitro",* and "protein expression *in vitro"* refer to a process characterised as an enzymatic production of polypeptides based on sequence of RNA or RNA analog in an artificial system. An *in vitro* system includes but is not limited to artificial reaction mixture, cellular lysates, nuclear lysates, cell cultures, and tissue cultures.

The terms "translation *in vivo",* "protein synthesis *in vivo",* and "protein expression *in vivo*" refer to a process characterised as an enzymatic production of polypeptides based on sequence of RNA or RNA analog in a natural system. An *in vivo* system includes but is not limited to whole organisms, organs, cellular and tissue transplants of human, animal, plant, fungal, or bacterial origin.

The terms "therapeutic protein" or "therapeutic proteins" refer to a polypeptide molecule that may be utilized in the course of therapy or medical studies. The classes of therapeutic proteins include but are not limited to antigens, antibodies, immunogenic proteins, fluorescent proteins, reporter proteins, protein hormones, membrane proteins, transcription factors, regulatory proteins, systems for gene editing and systems for gene regulation.

The terms "therapeutic gene" or "therapeutic genes" refer to a sequence of a nucleic acid containing an open-reading frame encoding the amino acid sequence of the therapeutic protein. Therapeutic genes may consist of but are not limited to sequences encoded in natural or synthetic viral DNA or RNA, plasmid vectors, RNA, mRNA or circRNA.

The term "pharmaceutical composition" refers to a composition of substances comprising of active substances and helper substances, such as pharmaceutically acceptable solvents and/or carriers. Pharmaceutical composition may be formulated as a solid, liquid, aqueous solution, aqueous emulsion, suspension, lipid nanoparticles, liposomes, or liposomal suspension. The term pharmaceutical composition includes but is not limited to compositions designed for expression of a therapeutic genes and proteins in *in vitro* and *in vivo* setup.

### Brief description of the drawings

The following detailed description of the embodiments of the invention will be better understood when read in conjunction with the appended drawings. For the purpose of illustrating the invention, there are shown in the drawings embodiments, which are presently exemplified. It should be understood, however, that the invention is not limited to the precise arrangement and instrumentalities of the embodiments shown in the drawings.

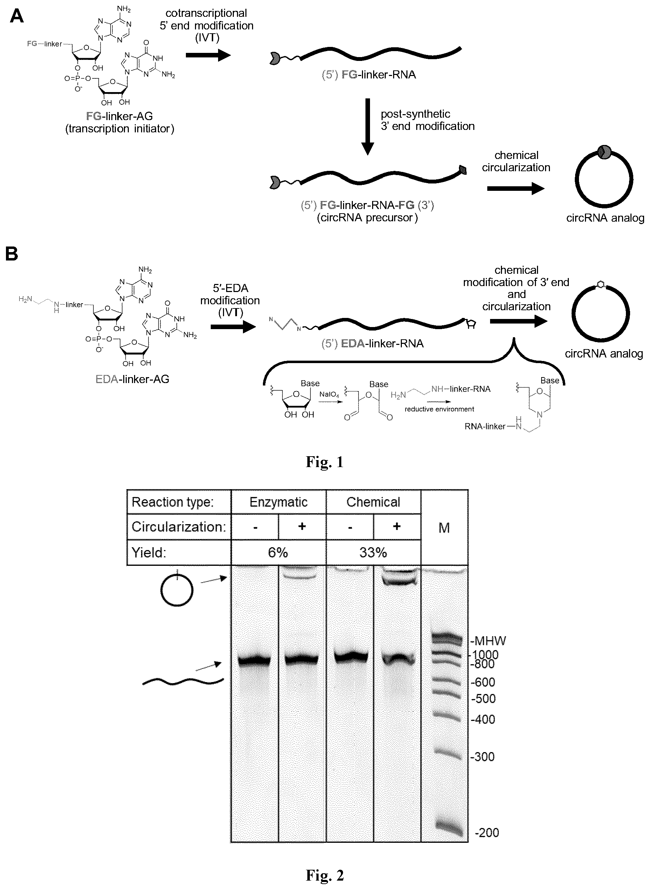

Figure 1 shows a general concept of preparation of circRNA analogs. A) First, the precursor of circRNA analog (FG-linker-RNA) is synthesized during in vitro transcription (IVT) reaction. Due to the presence of a transcription initiator (FG-linker-AG), the transcribed RNA contains a reactive functional grup (FG) at the 5' end. Next, the 3' end reactive functional group is incorporated into RNA post-synthetically, by either enzymatic reaction or, preferably, chemical reaction. The 5' and 3' reactive functional groups are reactive towards each other either spontaneously or in the presence of a specific trigger. B) As an example, a precursor of circRNA analog (EDA-linker-RNA) is synthesized during IVT. Due to the presence of a transcription initiator EDA-linker-AG, the transcribed RNA contains ethylenediamine (EDA) moiety at the 5' end. The precursor is first chemically modified at 3' end by treatment with periodate. This causes selective oxidation of the 3' ribose, which is prone to nucleophilic attack by the EDA moiety. Finally, the addition of a reductive agent (i. e. NaBH3CN) leads to an irreversible covalent linkage of RNA's 5' and 3' ends.

Figure 2 shows a comparison of enzymatic and chemical circularization. 5'-modified RNA (pre-circRNA2) was used as a precursor for chemical circularization. As a reference, 5'-monophosphorylated RNA of the same sequence (pre-circRNA1) was used as a substrate in enzymatic circularization catalyzed by T4 RNA ligase I. The substrates and products of chemical and enzymatic reactions were analyzed by PAGE (6% AA, AA:MBAA = 19:1, 7 M Urea, 1x TBE). The yields of the reactions were determined by densitometry. M refers to linear RNA length marker. HMW refers to linear RNA of length ranging from 1500 nt to 6000 nt.

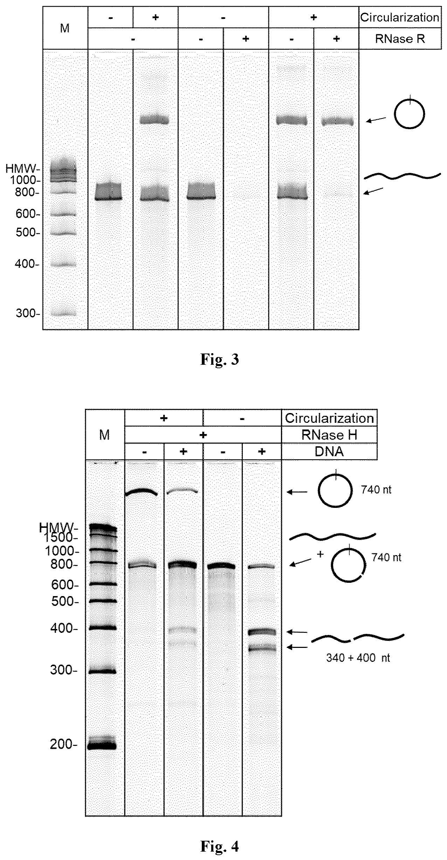

Figure 3 shows a RNase R digestion assay. Samples of a linear precursor of circRNA analog (pre-circRNA2) and products of chemical circularization (a mixture of pre-circRNA2 and circRNA2) were treated with a processive 3' exonuclease (RNase R), that selectively hydrolyses linear RNA species. After RNase R treatment the samples were analyzed by PAGE (6% AA, AA:MBAA = 38:1, 7 M Urea, 1x TBE). M refers to linear RNA length marker. HMW refers to linear RNA of length ranging from 2000 nt to 6000 nt.

Figure 4 shows a RNase H digestion assay. Samples of a circRNA analog (circRNA2) and its linear precursor (pre-circRNA2) underwent DNA-dependent endonucleolytic cleavage catalyzed by RNase H and subsequent PAGE. In presence of targeting DNA oligonucleotide, the circRNA analog is nicked. As a result, nicked circRNA exhibits electrophoretic mobility of a linear precursor. On the other hand, cleavage of the linear precursor leads to two RNA molecules of lower size. PAGE conditions: 6% AA, AA:MBAA = 38:1, 7 M Urea, 1x TBE. M refers to linear RNA length marker. HMW refers to linear RNA of length ranging from 2000 nt to 6000 nt.

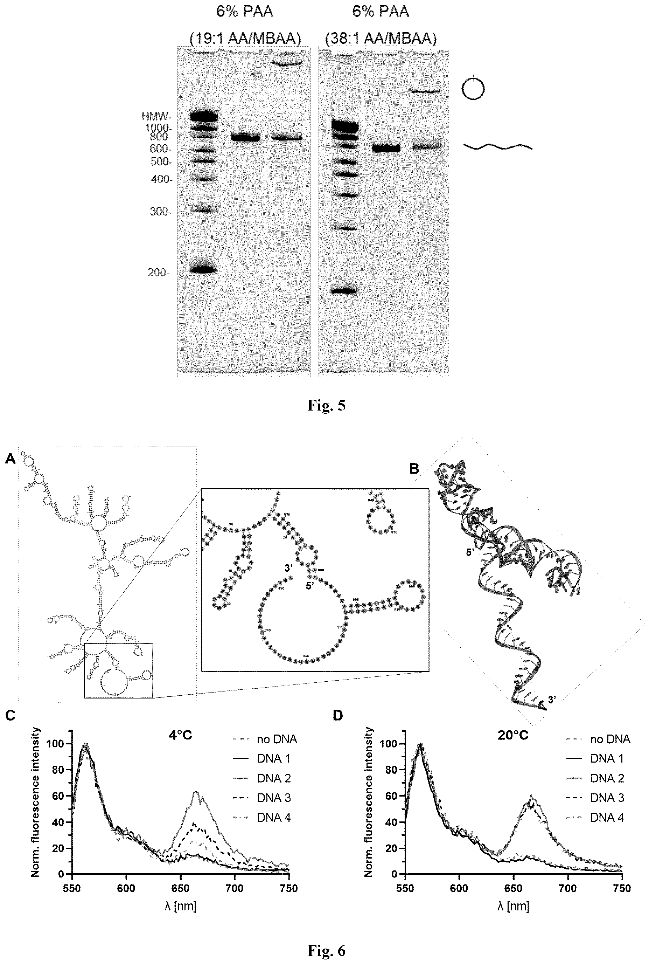

Figure 5 shows a polyacrylamide gel electrophoresis of circRNA - influence of gel crosslinking degree. Samples of a RNA length marker, a linear precursor of circRNA analog (pre-circRNA2), and products of chemical circularization (a mixture pre-circRNA2 and circRNA2) were analyzed by PAGE. Two types of 6% polyacrylamide gels were used in parallel: one with higher crosslinking degree (AA:MBAA = 19:1, 7 M Urea, 1x TBE) and one with lower crosslinking degree (AA:MBAA = 38:1, 7 M Urea, 1x TBE). The migration of circRNA is slower than that of linear RNA. Electrophoretic mobility differences are pronounced in gel with a higher crosslinking degree. HMW refers to linear RNA of length ranging from 2000 nt to 6000 nt.

Fig. 6 shows a optimizing RNA conformation using complementary DNA oligonucleotides. A) Prediction of minimum free energy structure of RNA5 sequence generated with RNAfold web server. B) Prediction of tertiary structure of an RNA sequence comprising flanking regions of RNA5 sequence generated with RNAComposer web server. The predictions indicate, that 3' poliA sequence may distance 5' and 3' ends and hinder any reaction between them. Fluorescence spectra of Cy5-RNA5-Cy3 probe recorded at 4°C (C) or 20°C (D) before (w/o ON) or after hybridization with of one of the complementary DNA oligonucleotides (ON2-ON5). Emergence of 660 nm emission band indicates the occurrence of the FRET phenomenon facilitated by decreased distance between 5' and 3' ends.

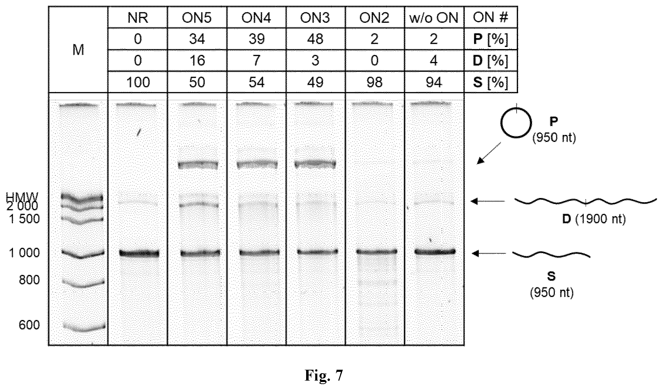

Fig. 7 shows a influence of complementary DNA oligonucleotides on chemical circularization. Pre-circRNA 10 was used as a precursor for chemical circularization. Before the oxidation step, the precursor was annealed with one of the complementary DNA oligonucleotides (ON2-ON5). After the reaction, the samples were analyzed by PAGE (6% AA, AA:MBAA = 38:1, 7 M Urea, 1x TBE). As a reference, samples of precursor before reaction (NR) and products of reaction without complementary DNA (w/o ON) were included. M refers to linear RNA length marker. HMW refers to linear RNA of length ranging from 3000 nt to 6000 nt. The relative amounts of product (P), linear dimer (D), and unreacted substrate (S) were determined by densitometry.

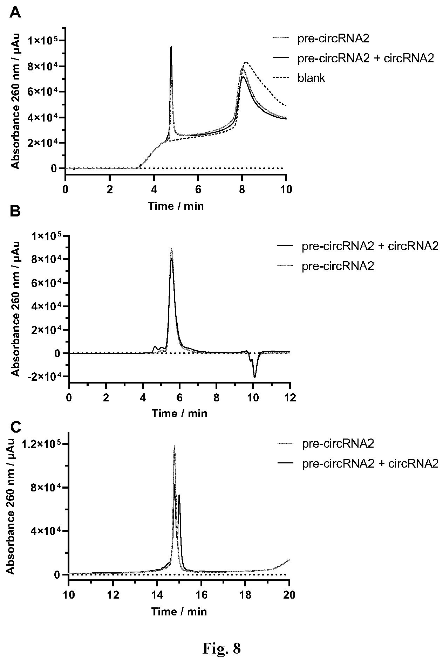

Fig. 8 shows an attempted separation of linear and circular RNA using IE-HPLC (A), SEC-HPLC (B) and RP-HPLC (C). A sample containing a mixture of linear and circular RNA (pre-circRNA2 + circRNA2) was chromatographed using three different modes and columns. As a reference, linear RNA (pre-circRNA2) was resolved in same conditions. IE-HPLC conditions: CIMac PrimaS^{™} 0.1 mL Analytical Column (2 µm); A - 50 mM HEPES pH 7.0; B - 200 mM sodium pyrophosphate, 50 mM HEPES pH 8.5; 0% B for 2 min, 0-100% B in 6 min, 100% B for 2 min, 100-0% B in 2 min, 0% B for 1 min; flow 1.0 ml/min at 25°C. SEC-HPLC conditions: Yarra 3 µm SEC-4000 300x7.8 mm; A - 100 mM Na2HPO4, 0.025% NaN3 pH 6.8; 100% A for 50 min; flow 1.2 ml/min at 25°C; RP-HPLC conditions: RNASept Prep C18 50x7.8 mm 2 µm; A - 100 mM HAA, 10% MeCN, pH 7.0; B - 100 mM HAA, 75% MeCN, pH 7.0; 20-70% B in 25 min; flow : 1.0 ml/min at 60 °C.

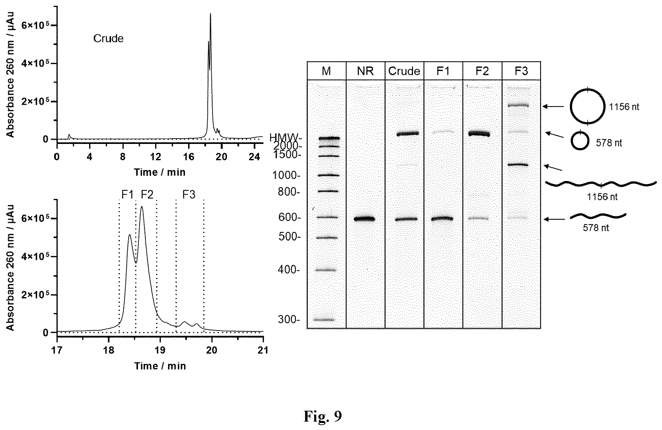

Figure 9 shows a reversed-phase high-pressure liquid chromatography of circRNA. Products of chemical circularization of pre-circRNA4 were chromatographed in denaturing conditions (60°C, hexylammonium acetate buffer and acetonitrile mixture as a mobile phase). The UV-absorbing eluate was collected and separated into fractions, that corresponded to different peaks of absorbance. Next, the fractions were concentrated and analyzed by PAGE, along with EP5-GLuc before and after circularization. NR - precursor (pre-circRNA4), Cr - crude products of circularization, F1-F3 - concentrated HPLC fractions, M - linear RNA length marker. HMW refers to linear RNA of length ranging from 3000 nt to 6000 nt. PAGE conditions: 6% AA, AA:MBAA = 38:1, 7 M Urea, 1x TBE.

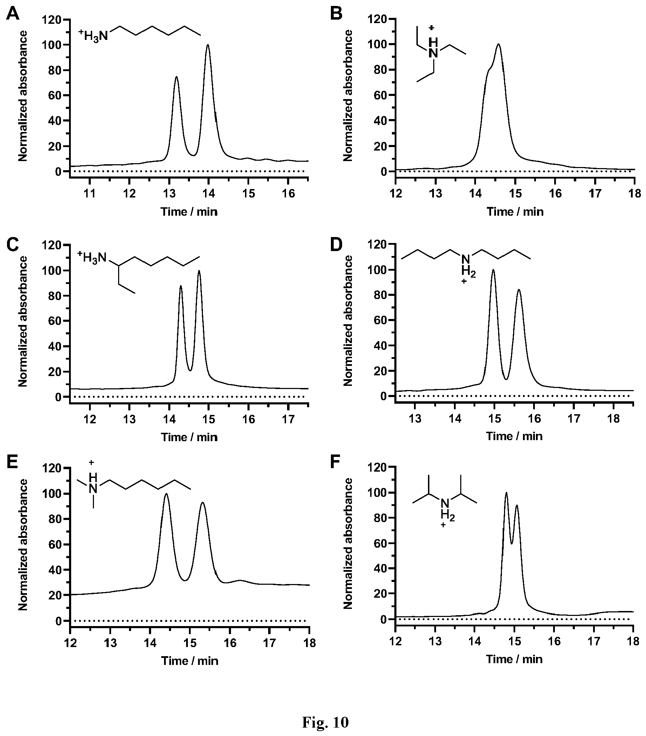

Figure 10 shows a influence of mobile phase composition on RP-HPLC of circRNA. A mixture of linear (pre-circRNA14) and circular (circRNA14) RNAs was resolved with RP-HPLC. Each chromatography was performed in presence of different ion-pairing agent: A) hexylammonium acetate; B) triethylammonium acetate; C) 2 ethylhexylammonium acetate; D) dibutylammonium acetate; E) N,N dimethylhexylammonium acetate; F) N,N diisopropylammonium acetate.

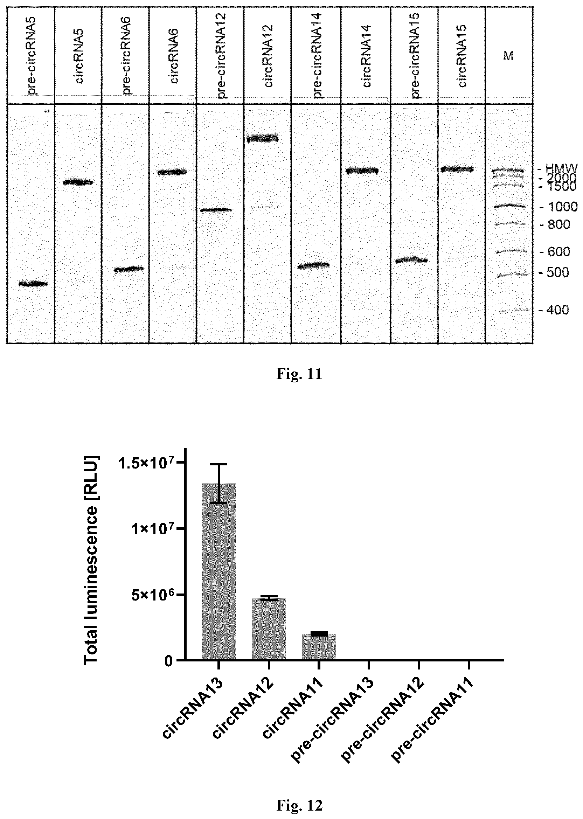

Figure 11 shows a PAGE of selected circRNA precursors and circRNA after HPLC and RNase R treatment. M - linear RNA length marker. HMW refers to linear RNA of length ranging from 3000 nt to 6000 nt. PAGE conditions: 6% AA, AA:MBAA = 38:1, 7 M Urea, 1x TBE.

Figure 12 shows a translation of circRNA analogs in vitro. HEK293 cells were transfected with unmodified (circRNA11) or modified circRNA (circRNA12 with unnatural linkage, and circRNA13 with a cap structure) encoding Gaussia luciferase, a reporter protein secreted by the cells to the medium. The circRNA precursors (pre-circRNA11-13) were used as a reference. The cell media were harvested at timepoints (4, 24, 48, 72, 96, 120, 144 and 168 hours post transfection) and used for quantification of the reporter protein levels with luciferase bioluminescence assay. The detected luminescence from all timepoints was summarized to give the total luminescence. The data presented in the graph derives from tree independent repetitions, error bars represent a standard error of the mean (SEM).

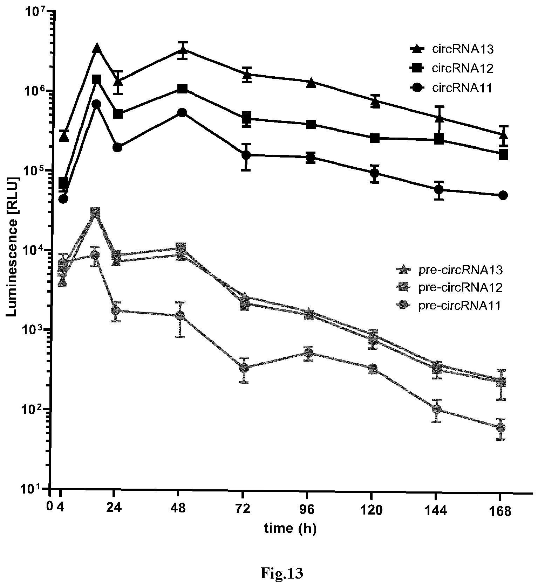

Figure 13 shows a translation of circRNA analogs in vitro (continued). HEK293 cells were transfected with unmodified (circRNA11) or modified circRNA (circRNA12, 13) encoding Gaussia luciferase, a reporter protein secreted by the cells to the medium. The circRNA precursors (pre-circRNA11-13) were used as a reference. The cell media were harvested at timepoints (4, 24, 48, 72, 96, 120, 144 and 168 hours post transfection) and used for quantification of the reporter protein levels with luciferase bioluminescence assay. The data presented in the graph derives from tree independent repetitions, error bars represent a standard error of the mean (SEM).

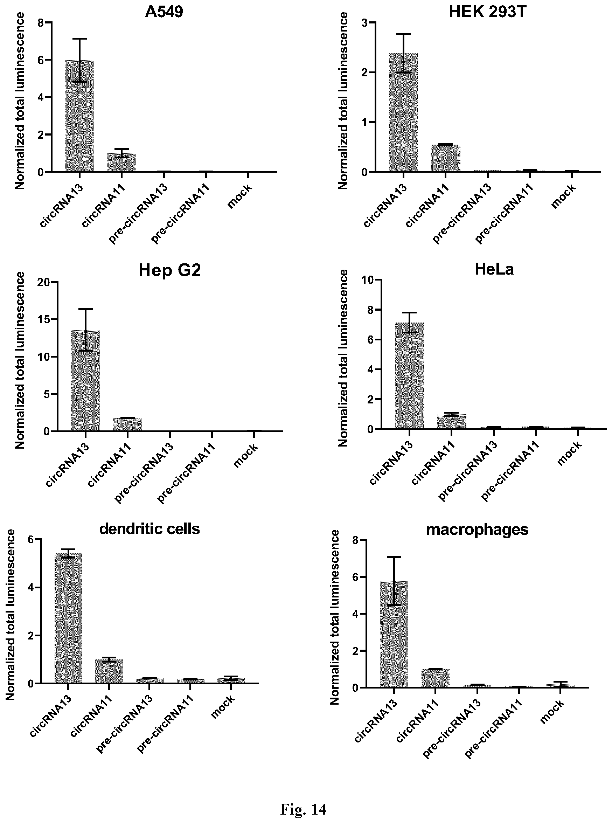

Figure 14 shows a translation of circRNA analogs in vitro. HEK 293T, Hep G2, A549, HeLa, mouse dendritic cells as well as macrophages (established from bone marrow monocytes) were transfected with unmodified (circRNA11) or modified circRNA (circRNA13) encoding Gaussia luciferase, a reporter protein secreted by the cells to the medium. The circRNA precursors (pre-circRNA11 and pre-circRNA13) as well as mock transfection (water instead of RNA) were used as a reference. The cell media were harvested at timepoints (4, 24, 48, 72, 96, 120, 144 and 168 hours post transfection or 4, 24, 48, 72, 96 and 120 hours post transfection as in case of dendritic cells) and used for quantification of the reporter protein levels with luciferase bioluminescence assay. The detected luminescence from all timepoints was summarized to give the total luminescence, that was subsequently normalizes to the average luminescence obtained for circRNA11. The data presented in the graph derives from tree independent repetitions, error bars represent a standard error of the mean (SEM).

### Detailed description

The present invention relates to circular RNA (circRNA) analogs obtained by circularization of a circRNA precursor containing two reactive functional groups, one at the 5'-end and a second at the 3' end. [Fig.1A] The reactive functional group at the 5'-end is incorporated into RNA during enzymatic synthesis (i.e. *in vitro* transcription reaction). The 3'-end reactive functional group is incorporated into RNA post-synthetically, by either enzymatic reaction or, preferably, chemical reaction. The 5' and 3' reactive groups are reactive towards each other either spontaneously or in the presence of a specific trigger, such as addition of a chemical reagent, addition of a catalysts, change of temperature, presence of light, change of pH of the solution, etc. The representative pairs of 5' and 3' reactive groups reactive towards each other include, but are not limited to: EDA and dialdehyde, hydrazide and dialdehyde, azide and alkyne, azide and cyclooctyne derivative, amino and carboxylic group, amino and fluorosulfonylgroup, azide and phosphine, etc..

A representative example of functional group at the 5'-end is EDA motif (EDA-linker-RNA). [Fig.1B] The precursor is a modified nucleic acid, that can be prepared by *in vitro* transcription. The 5'-EDA motif can be readily installed during the in vitro transcription (IVT) reaction if the transcription is carried out in the presence of an EDA-modified transcription initiator [see Table 1 for examples]. This is beneficial, as unlike other methods of chemical circularization, the precursor's length is not limited by the efficiency of RNA solid-phase synthesis. The 5'-EDA motif and the nucleic acid chain of the EDA-linker-RNA are joined by a linker. The linker itself may be a linear or branched combination of simple chemical moieties (such as carbohydrate or PEG linkers), or it may contain more complex elements, such as detectable labels or a 7-methylguanosine cap analog. The circRNA precursor can bear additional modifications, such as detectable labels or 7-methylguanosine cap analogs, as long as they do not interfere with the circularization reaction. This is beneficial, as in contrast to enzymatic circularization, the user is not restricted by the activity and substrate-specificity of a particular enzyme. The chemical circularization reaction is a two-step process. First, the circRNA precursor is selectively oxidized with periodate at the 3' end to introduce a reactive functional group (a dialdehyde) at the 3' end of RNA. This occurs via ring-opening oxidation and formation of a reactive acyclic dialdehyde derivative of the terminal 3'-end ribose. During the second step, the 3'-end dialdehyde derivative spontaneously reacts with the 5' EDA motif. This step requires reductive environment for optimal performance. If reductive environment is ensured during this reaction, the reduction of the formed intermediate occurs making the whole process irreversible. Except the presence of 5' EDA motif (5' reactive group) and 3' dialdehyde motif (3' reactive group), the chemical circularization has no requirements concerning chemical structure or the sequence of the circRNA precursor. This is beneficial, as in contrast to circularization techniques based on activity of catalytic nucleic acids (PIE and Tornado systems), the invented chemical circularization does not require sequence engineering and construct cloning for preparation of the circRNA precursor.

circRNA precursors containing EDA motif were prepared by means of IVT and subsequently circularized [Table 1]. As a comparison, precursors bearing 5' monophosphate moiety and identical sequence were subjected to enzymatic circularization catalysed by T4 RNA ligase I or T4 RNA ligase II. Unexpectedly, the yield of chemical circularization in comparison to enzymatic circularization is less dependent on the precursor sequence and is often more efficient [Fig.2].

The circular topology of the circRNA analogs obtained by chemical circularization can be verified with exonucleolytic digestion assays, endonucleolytic assays, as well as electrophoretic mobility assays.

Exonucleolytic digestion assays allow one to distinguish linear RNAs from circular RNAs, as exonucleases can hydrolyse only RNA molecules that contain free 5' or 3' end. On the other hand, circular RNAs are resistant towards exonucleolytic hydrolysis as it lacks free 5' and 3' ends. RNase R is a processive 3' exonuclease, that is most commonly used for such assay.³ The products of chemical circularization were treated with RNase R [Fig.3]. In contrary to the precursors, the circRNA analogs were resistant to RNase R hydrolysis.

Endonucleolytic assays are based on a phenomenon of nicking and linearization of circRNA. A site-selective and sequence-dependent cleavage of a circular RNA molecule leads to a single linear RNA molecule of equal size. On the other hand, cleavage of a linear RNA molecule leads to two linear RNA molecules of smaller sizes. Such assay can be performed with RNase H, an endonuclease, that nicks RNA in RNA-DNA duplexes.²⁵ RNase H and targeting DNA oligonucleotides were used for nicking of the products of chemical circularization [Fig. 4]. The assay confirmed that, in contrast to the precursors, nicking of the circRNA analogs leads to their linearisation.

Electrophoretic mobility of nucleic acids is affected by their size, charge, conformation, and topology.^{26, 27} Thanks to that, circRNA and their precursors can be separated during polyacrylamide gel electrophoresis in denaturing conditions [Fig. 5]. The crosslinking degree of the polyacrylamide gel matrix is a crucial factor for separation of RNA based on their topology. The electrophoretic separation of linear and circular RNA using a cross-linked polyacrylamide gel is significantly better than with regular or commercially available agarose gels.²⁷

The circularization efficiency is affected by the sequence of the circRNA precursor. [Tab. 2] In some cases, such as sequences containing a 3' poliA sequence, the circularization yield can be increased by addition of a complementary nucleic acid oligomer. [Tab. 3] During the reaction, the oligomer and precursor hybridize and affect the conformation of the precursor. Using an oligomer with an optimal design, the conformational change of the precursor decreases the distance between 5' and 3' ends [Fig. 6]. In consequence, the optimal distance and orientation of the 5' and 3' ends increase the circularization yield [Fig. 7].

The present invention also relates to methods of preparation and isolation of circRNA analogs. This includes development of chromatographic methods of linear and circular RNA resolution.

Previously reported methods of chromatography of circularization products were based on size-exclusion chromatography in HPLC setup (SEC-HPLC).^{11, 12, 14} This method allows one to separate sample components based on hydrodynamic radius and is considered to provide one of the mildest chromatographic conditions. SEC-HPLC was used to resolve the products of PIE-based circularization.^{11, 12, 14} In these cases, the precursor and circRNA significantly differ in molecular size between each other, as well as form side products (such as free introns). In case of synthetic polymers (such as polystyrene or poli(ethyneneoxide)) the hydrodynamic radius (Stokes radius) is decreased upon circularization.²⁸ This property was used to separate linear and cyclic polymers using gel permeation chromatography (GPC). However, the decrease of the Stokes radius of an RNA molecule caused by its circularization have not been documented. One could speculate, that intramolecular interactions, that contribute to existence of a landscape of RNA conformers, may diminish the influence of topological change on differentiation of Stokes radii of circular and linear RNA.²⁹ This could also explain why SEC-HPLC in mild conditions leads to coelution of intact and nicked circRNA.^{12, 14}

During the development of the invention, it was found that both SEC-HPLC and ion-exchange HPLC (IE-HPLC) are insufficiently effective for separation of linear and circular RNA of the same molar mass [Fig. 8]. The reversed-phase HPLC (RP-HPLC) in ion-pair mode was found to be the most suitable method for separation of circularization products [Fig. 8, 9]. Chromatography was performed using polistyren-divinylbenzen (PSDVB) resins, elevated temperature (50-80 °C), and mixture of aqueous solution of ion pair reagent with acetonitrile as a mobile phase. The separation of linear and circular RNA is affected by composition of the mobile phase, namely the chosen ion pair reagent. The most effective resolution was obtained with 100 mM n-hexylammonium acetate (HAA), with linear gradient of acetonitrile (45-58,5% in 25 min, flowrate 1.00 ml/min at 60 °C) [Fig. 10].

The present invention also relates to therapeutic application of circRNA analogs. To demonstrate the applicability of protein-encoding circRNA analogs in gene delivery, circRNA analogs encoding reporter protein, *Gaussia* luciferase, were synthesized and expressed in cells. The levels of the reporter protein were higher in case of expression of chemically modified circRNA analogs (circRNA12, 13) than unmodified circRNA (circRNA11) [Fig. 12]. Both modified and unmodified circRNAs exhibited increased duration of protein expression in comparison to their linear precursors (pre-circRNA11-13) [Fig. 13]. This result shows that chemical modification within the circRNA analogs is beneficial in the context of therapeutic protein expression.

### Tables and their brief descriptions

**Table 1: List of DNA templates (linearized plasmids) and corresponding RNA sequences with description and properties. Column description: # plasmid - designation of a plasmid; Restrictase - name of the enzyme used for linearization of the plasmid; # RNA sequence - designation of a RNA sequence encoded in the DNA template for IVT; RNA sequence elements - 5' and 3' untranslated regions (5' UTR and 3' UTR), protein-coding sequence (CDS), sequence downstream of the 3' UTR (3' tail); RNA length - total length of the transcript; RNA E₂₆₀ - molar extinction coefficient calculated for particular RNA sequence.³⁰ HBB: human betaglobin UTR (5' or 3') sequence, EMCV: Encephalomyocarditis virus IRES, RSV: respiratory syncytial virus IRES, OligoIRES: synthetic IRES, eGFP: enhanced green fluorescent protein sequence, GLuc: Gaussia-Dura luciferase sequence, A30: 30 nucleotide-long poliadenine sequence.**

| # Plasmid | Restrictase | # RNA sequence | RNA sequence elements | | | | RNA length [nt] | RNA E₂₆₀ [µM⁻¹-cm⁻¹] |
|---|---|---|---|---|---|---|---|---|
| | | | 5' UTR | CDS | 3' UTR | 3' tail | | |
| PL1 | BarnHI | RNA1 | - | eGFP | - | - | 740 | 8.93 |
| PL2 | BarnHI | RNA2 | - | GLuc | - | - | 578 | 6.99 |
| PL3 | BarnHI | RNA3 | HBB | | - | - | 626 | 7.58 |
| | PmeI | RNA4 | | | HBBx2 | - | 918 | 11.0 |
| | AarI | RNA5 | | | HBBx2 | A30 | 952 | 11.5 |
| PL4 | BarnHI | RNA6 | EMCV | | - | - | 1152 | 13.9 |
| PL5 | | RNA7 | RSV | | - | - | 598 | 7.25 |
| PL6 | | RNA8 | OligoIRES | | - | - | 711 | 9.35 |

**Table 2: List of circRNA and yields of their chemical or enzymatic circularization. Column description: # circRNA - designation of a circRNA and corresponding pre-circRNA; # RNA sequence - designation of an RNA sequence encoded in the DNA template for IVT; IVT initiator - compound used as transcription initiator in IVT reaction; Circularization type - type of reaction (chemical or enzymatic catalyzed by T4RNA ligase I or T4 RNA ligase II) used for RNA circularization; DNA oligonucleotide - information on whether a complementary DNA oligonucleotide was used during particular circularization reaction; Circularization yield - fraction of the circRNA precursor converted to particular circRNA product during circularization reaction, determined by PAGE of crude circularization products and densitometric analysis.**

| # circRNA | # RNA sequence | IVT initiator | Circularization type | DNA oligonucleotide | Circularization yield [%] |
|---|---|---|---|---|---|
| circRNA1 | RNA1 | GMP | Enz. (T4 Lig. 1) | w/o ON | 5-10 |
| circRNA2 | | EDA- PEG5-AG | Chem. | | 30-45 |
| circRNA3 | RNA2 | GMP | Enz. (T4 Lig. 1) | | 36 |
| circRNA4 | | EDA- PEG5-AG | Chem. | | 60 |
| circRNA5 | | EDA- PEG5- Cap1 | | | 53 |
| circRNA6 | RNA3 | [?] EDA- PEG5- Cap1 | Chem. | | 46 |
| circRNA7 | RNA4 | GMP | Enz. (T4 Lig. 1) | w/o ON | 13 |
| | | | Enz. (T4 Lig. 2) | w/o ON | 31 |
| | | | | ON1 | 1-45 |
| circRNA8 | | EDA- PEG5- Cap1 | Chem. | w/o ON | 10 |
| | | | | ON1 | 50 |
| circRNA9 | RNA5 | GMP | Enz. (T4 Lig. 1) | w/o ON | 9 |
| | | | Enz. (T4 Lig. 2) | w/o ON | 32 |
| | | | | ON4 | 61 |
| circRNA 10 | | EDA- PEG5- Cap1 | Chem | w/o ON | 2 |
| | | | | ON4 | 40 |
| circRNA 11 | RNA6 | GMP | Enz. (T4 Lig. 1) | w/o ON | 10 |
| | | | Enz. (T4 Lig. 2) | ON6 | 46 |
| circRNA12 | | EDA- PEG5-AG | Chem | w/o ON | 44 |
| | | | | ON6 | 44 |
| circRNA13 | | EDA- PEG5- Cap1 | | ON6 | 41 |
| circRNA14 | RNA7 | GMP | Enz. (T4 Lig. 1) | w/o ON | 40 |
| | | | Enz. (T4 Lig. 2) | | 84 |
| circRNA15 | | EDA- PEG5-AG | Chem | | 52 |
| circRNA16 | RNA8 | GMP | Enz. (T4 Lig. 1) | | 5-10 |
| circRNA17 | | EDA- PEG5-AG | Chem | | 53 |

**Table 3: List of complementary DNA oligonucleotides used for RNA circularization and FRET experiments. Column descriptions: #ON - designation of a DNA oligonucleotide sequence; # RNA sequence - designation of an RNA sequence; ON sequence - sequence of the designated oligonucleotide.**

| # ON | # RNA sequence | ON sequence |
|---|---|---|
| ON1 | RNA4 | AGCAAATGTCTAGATTATCCCTAACATTTAAATGCAATGAAAA |
| ON2 | RNA5 | TCCCTTTTTT |
| ON3 | | TCTAGATTATCCCTTTTTTTTTTTTTTT |
| ON4 | | GAAGCAAATGTCTAGATTATCCCTTTTTTTTTTTTTTTTTTTTTTTTT |
| ON5 | | GAAGCAAATGTCTAGATTATCCCTTTTTTTTTTTTTTTTTTTTTTTTTTTTTTTGTTTAAACATTTA |
| ON6 | RNA6 | AGGGGGGGAGGGAGAGGGGTACCCCCTATCCTTAGTCACCACCGGCCCCCT |

**Table 4: List of plasmids and inserted sequences, used for preparation of IVT templates. Underlined is the coding sequence from the T7 RNA polymerase transcription start site to the site of linearization. The recognition sequence for BarnHI (GGATCC), PmeI (GTTTAAAC) and AarI (AAAAAAAAAGCAGGTG) are in bold with the cut site between nucleotides in italic.**

| | |
|---|---|
| PL1 | GCCGAATTATATTATTTTTGCCAAATAATTTTTAACAAAAGCTCTGAAGTCTTCTTCATTTAAATTC TTAGATGATACTTCATCTGGAAAATTGTCCCAATTAGTAGCATCACGCTGTGTAATACGACTCACT ATAGGGGGTACGCCACCATGGTGAGCAAGGGCGAGGAGCTGTTCACCGGGGTGGTGCCCATCCTG GTCGAGCTGGACGGCGACGTAAACGGCCACAAGTTCAGCGTGTCCGGCGAGGGCGAGGGCGATGC CACCTACGGCAAGCTGACCCTGAAGTTCATCTGCACCACCGGCAAGCTGCCCGTGCCCTGGCCCAC CCTCGTGACCACCCTGACCTACGGCGTGCAGTGCTTCAGCCGCTACCCCGACCACATGAAGCAGCA CGACTTCTTCAAGTCCGCCATGCCCGAAGGCTACGTCCAGGAGCGCACCATCTTCTTCAAGGACGA CGGCAACTACAAGACCCGCGCCGAGGTGAAGTTCGAGGGCGACACCCTGGTGAACCGCATCGAGC TGAAGGGCATCGACTTCAAGGAGGACGGCAACATCCTGGGGCACAAGCTGGAGTACAACTACAAC AGCCACAACGTCTATATCATGGCCGACAAGCAGAAGAACGGCATCAAGGTGAACTTCAAGATCCG CCACAACATCGAGGACGGCAGCGTGCAGCTCGCCGACCACTACCAGCAGAACACCCCCATCGGCG |
| | ACGGCCCCGTGCTGCTGCCCGACAACCACTACCTGAGCACCCAGTCCGCCCTGAGCAAAGACCCC AACGAGAAGCGCGATCACATGGTCCTGCTGGAGTTCGTGACCGCCGCCGGGATCACTCTCGGCAT GGACGAGCTGTACAAGTAAGGATCCTAGGGCCGCAGCTCGCTTTCTTGCTGTCCAATTTCTATTAA AGGTTCCTTTGTTCCCTAAGTCCAACTACTAAACTGGGGGATATTATGAAGGGCCTTGAGCATTTG GATTCTGCCTAATAAAAAACATTTATTTTCATTGCGTTTAGCTCGCTTTCTTGCTGTCCAATTTCTAT TAAAGGTTCCTTTGTTCCCTAAGTCCAACTACTAAACTGGGGGATATTATGAAGGGCCTTGAGCAT TTGGATTCTGCCTAATAAAAAACATTTATTTTCATTGCATTTAAATGTTTAAACAAAAAAAAAAAAA AAAAAAAAAAAAAAAAAAAAAAAAAAAAAAAAAAAAAAAAAAAAAAAAAAAAAAAAAAAAAAAAAA AAAAAAAAAAAAAAAAAAAAAAAAAAAAAAAAAAAAAAAAAAAAAAAAAAAAAAAAGCAGGTGG GAGGGTACGTTCGTGATAAACAAAC |
| PL2 | GCCCCTGCAGCCGAATTATATTATTTTTGCCAAATAATTTTTAACAAAAGCTCTGAAGTCTTCTTCA TTTAAATTCTTAGATGATACTTCATCTGGAAAATTGTCCCAATTAGTAGCATCACGCTGTGTAATAC GACTCACTATAGGGGGTACGCCACCATGGGAGTCAAAGTTCTGTTTGCCCTGATCTGCATCGCTGT GGCCGAGGCCAAGCCCACCGAGAACAACGAAGACTTCAACATCGTGGCCGTGGCCAGCAACTTCG CGACCACGGATCTCGATGCTGACCGCGGGAAGTTGCCCGGCAAGAAGCTGCCGCTGGAGGTGCTC AAAGAGTTGGAAGCCAATGCCCGGAAAGCTGGCTGCACCAGGGGCTGTCTGATCTGCCTGTCCCA CATCAAGTGCACGCCCAAGATGAAGAAGTTCATCCCAGGACGCTGCCACACCTACGAAGGCGACA AAGAGTCCGCACAGGGCGGCATAGGCGAGGCGATCGTCGACATTCCTGAGATTCCTGGGTTCAAG GACTTGGAGCCCTTGGAGCAGTTCATCGCACAGGTCGATCTGTGTGTGGACTGCACAACTGGCTGC CTCAAAGGGCTTGCCAACGTGCAGTGTTCTGACCTGCTCAAGAAGTGGCTGCCGCAACGCTGTGCG ACCTTTGCCAGCAAGATCCAGGGCCAGGTGGACAAGATCAAGGGGGCCGGTGGTGACTAAGGAT CCTAGGGCCGCAGCTCGCTTTCTTGCTGTCCAATTTCTATTAAAGGTTCCTTTGTTCCCTAAGTCCA ACTACTAAACTGGGGGATATTATGAAGGGCCTTGAGCATTTGGATTCTGCCTAATAAAAAACATTT ATTTTCATTGCGTTTAGCTCGCTTTCTTGCTGTCCAATTTCTATTAAAGGTTCCTTTGTTCCCTAAGT CCAACTACTAAACTGGGGGATATTATGAAGGGCCTTGAGCATTTGGATTCTGCCTAATAAAAAACA TTTATTTTCATTGCATTTAAATGTTTAAACAAAAAAAAAAAAAAAAAAAAAAAAAAAAAAAAAA AAAAAAAAAAAAAAAAAAAAAAAAAAAAAAAAAAAAAAAAAAAAAAAAAAAAAAAAAAAAAA AAAAAAAAAAAAAAAAAAAAAAAAAAAAAAAAAAAAGCAGGTGGGAGGGTACGTTCGTGATAAACAAA C |
| PL3 | CTGCAGCCGAATTATATTATTTTTGCCAAATAATTTTTAACAAAAGCTCTGAAGTCTTCTTCATTTA AATTCTTAGATGATACTTCATCTGGAAAATTGTCCCAATTAGTAGCATgcCACGCTGTGTAATACGA CTCACTATAGGGATAATctagacatttgcttctgacacaactgtgttcactagcaacctcaaacagacaccATGGGAGTCAAAGTTC TGTTTGCCCTGATCTGCATCGCTGTGGCCGAGGCCAAGCCCACCGAGAACAACGAAGACTTCAACA TCGTGGCCGTGGCCAGCAACTTCGCGACCACGGATCTCGATGCTGACCGCGGGAAGTTGCCCGGC AAGAAGCTGCCGCTGGAGGTGCTCAAAGAGTTGGAAGCCAATGCCCGGAAAGCTGGCTGCACCAG GGGCTGTCTGATCTGCCTGTCCCACATCAAGTGCACGCCCAAGATGAAGAAGTTCATCCCAGGACG CTGCCACACCTACGAAGGCGACAAAGAGTCCGCACAGGGCGGCATAGGCGAGGCGATCGTCGACA TTCCTGAGATTCCTGGGTTCAAGGACTTGGAGCCCTTGGAGCAGTTCATCGCACAGGTCGATCTGT GTGTGGACTGCACAACTGGCTGCCTCAAAGGGCTTGCCAACGTGCAGTGTTCTGACCTGCTCAAGA AGTGGCTGCCGCAACGCTGTGCGACCTTTGCCAGCAAGATCCAGGGCCAGGTGGACAAGATCAAG GGGGCCGGTGGTGACTAAGGATCCTAGGGCCGCAGCTCGCTTTCTTGCTGTCCAATTTCTATTAAA GGTTCCTTTGTTCCCTAAGTCCAACTACTAAACTGGGGGATATTATGAAGGGCCTTGAGCATTTGG ATTCTGCCTAATAAAAAACATTTATTTTCATTGCGTTTAGCTCGCTTTCTTGCTGTCCAATTTCTATT AAAGGTTCCTTTGTTCCCTAAGTCCAACTACTAAACTGGGGGATATTATGAAGGGCCTTGAGCATT TGGATTCTGCCTAATAAAAAACATTTATTTTCATTGCATTTAAATGTTTAAACAAAAAAAAAAAA AAAAAAAAAAAAAAAAAAAAAAGCAGGTGGGAGGGTACGTTCGTGATAAACAAACGGCCGC |
| PL4 | CCTGCAGCCGAATTATATTATTTTTGCCAAATAATTTTTAACAAAAGCTCTGAAGTCTTCTTCATTT AAATTCTTAGATGATACTTCATCTGGAAAATTGTCCCAATTAGTAGCATgCCACGCTGTGTAATACG ACTCACTATAGGGGGTACCCCTCTCCCTCCCCCCCTAACGTTACTGGCCGAAGCCGCTTGGAATAA GGCCGGTGTGCGTTTGTCTATATGTTATTTTCCACCATATTGCCGTCTTTTGGCAATGTGAGGGCCC GGAAACCTGGCCCTGTCTTCTTGACGAGCATTCCTAGGGGTCTTTCCCCTCTCGCCAAAGGAATGC AAGGTCTGTTGAATGTCGTGAAGGAAGCAGTTCCTCTGGAAGCTTCTTGAAGACAAACAACGTCTG TAGCGACCCTTTGCAGGCAGCGGAACCCCCCACCTGGCGACAGGTGCCTCTGCGGCCAAAAGCCA CGTGTATAAGATACACCTGCAAAGGCGGCACAACCCCAGTGCCACGTTGTGAGTTGGATAGTTGTG GAAAGAGTCAAATGGCTCTCCTCAAGCGTATTCAACAAGGGGCTGAAGGATGCCCAGAAGGTACC CCATTGTATGGGATCTGATCTGGGGCCTCGGTGCACATGCTTTACATGTGTTTAGTCGAGGTTAAA AAACGTCTAGGCCCCCCGAACCACGGGGACGTGGTTTTCCTTTGAAAAACACGATGATAATATGGC CACACATATGGGAGTCAAAGTTCTGTTTGCCCTGATCTGCATCGCTGTGGCCGAGGCCAAGCCCAC CGAGAACAACGAAGACTTCAACATCGTGGCCGTGGCCAGCAACTTCGCGACCACGGATCTCGATG CTGACCGCGGGAAGTTGCCCGGCAAGAAGCTGCCGCTGGAGGTGCTCAAAGAGTTGGAAGCCAAT GCCCGGAAAGCTGGCTGCACCAGGGGCTGTCTGATCTGCCTGTCCCACATCAAGTGCACGCCCAAG ATGAAGAAGTTCATCCCAGGACGCTGCCACACCTACGAAGGCGACAAAGAGTCCGCACAGGGCGG CATAGGCGAGGCGATCGTCGACATTCCTGAGATTCCTGGGTTCAAGGACTTGGAGCCCTTGGAGCA GTTCATCGCACAGGTCGATCTGTGTGTGGACTGCACAACTGGCTGCCTCAAAGGGCTTGCCAACGT GCAGTGTTCTGACCTGCTCAAGAAGTGGCTGCCGCAACGCTGTGCGACCTTTGCCAGCAAGATCCA GGGCCAGGTGGACAAGATCAAGGGGGCCGGTGGTGACTAAGGATCCTAGGGCCGCAGCTCGCTTT CTTGCTGTCCAATTTCTATTAAAGGTTCCTTTGTTCCCTAAGTCCAACTACTAAACTGGGGGATATT |
| | ATGAAGGGCCTTGAGCATTTGGATTCTGCCTAATAAAAAACATTTATTTTCATTGCGTTTAGCTCGC TTTCTTGCTGTCCAATTTCTATTAAAGGTTCCTTTGTTCCCTAAGTCCAACTACTAAACTGGGGGAT ATTATGAAGGGCCTTGAGCATTTGGATTCTGCCTAATAAAAAACATTTATTTTCATTGCATTTAAAT GTTTAAACAAAAAAAAAAAAAAAAAAAAAAAAAAAAAAAAAAGCAGGTGGGAGGGTACGTTCG TGATAAACAAACGGCCGCT |
| PL5 | CCTGCAGCCGAATTATATTATTTTTGCCAAATAATTTTTAACAAAAGCTCTGAAGTCTTCTTCATTT AAATTCTTAGATGATACTTCATCTGGAAAATTGTCCCAATTAGTAGCATGCCACGCTGTGTAATAC GACTCACTATAGGGGGTAATGAGTCCTGGACTGAAACGGACTCATATGGGAGTCAAAGTTCTGTTT GCCCTGATCTGCATCGCTGTGGCCGAGGCCAAGCCCACCGAGAACAACGAAGACTTCAACATCGT GGCCGTGGCCAGCAACTTCGCGACCACGGATCTCGATGCTGACCGCGGGAAGTTGCCCGGCAAGA AGCTGCCGCTGGAGGTGCTCAAAGAGTTGGAAGCCAATGCCCGGAAAGCTGGCTGCACCAGGGGC TGTCTGATCTGCCTGTCCCACATCAAGTGCACGCCCAAGATGAAGAAGTTCATCCCAGGACGCTGC CACACCTACGAAGGCGACAAAGAGTCCGCACAGGGCGGCATAGGCGAGGCGATCGTCGACATTCC TGAGATTCCTGGGTTCAAGGACTTGGAGCCCTTGGAGCAGTTCATCGCACAGGTCGATCTGTGTGT GGACTGCACAACTGGCTGCCTCAAAGGGCTTGCCAACGTGCAGTGTTCTGACCTGCTCAAGAAGTG GCTGCCGCAACGCTGTGCGACCTTTGCCAGCAAGATCCAGGGCCAGGTGGACAAGATCAAGGGGG CCGGTGGTGACTAAGGATCCTAGGGCCGCAGCTCGCTTTCTTGCTGTCCAATTTCTATTAAAGGTT CCTTTGTTCCCTAAGTCCAACTACTAAACTGGGGGATATTATGAAGGGCCTTGAGCATTTGGATTCT GCCTAATAAAAAACATTTATTTTCATTGCGTTTAGCTCGCTTTCTTGCTGTCCAATTTCTATTAAAG GTTCCTTTGTTCCCTAAGTCCAACTACTAAACTGGGGGATATTATGAAGGGCCTTGAGCATTTGGA TTCTGCCTAATAAAAAACATTTATTTTCATTGCATTTAAATGTTTAAACAAAAAAAAAAAAAAAAA AAAAAAAAAAAAAAAAAGCAGGTGGGAGGGTACGTTCGTGATAAACAAACGGCCGCT |
| PL6 | GCCCCTGCAGCCGAATTATATTATTTTTGCCAAATAATTTTTAACAAAAGCTCTGAAGTCTTCTTCA TTTAAATTCTTAGATGATACTTCATCTGGAAAATTGTCCCAATTAGTAGCATgCCACGCTGTGTAAT ACGACTCACTATAGGGGGTAGGCGCGCCAGTCCTTCTGTAGCGACCCTTTGCAGGCAGCGGAACCC CCCACCTGGCGACAGGTGCCTCTGCGGCCAAAAGCCACGTGTATAAGATACACCTGCAAAGGCGG CACAACCCCAGTGCCACGTTGTGAGTTGGATAGTTGTGGAAAGAGTCAAATGGCTCTCCTCAAGCG TATTCAACAAGCGACTCGGACATATGGGAGTCAAAGTTCTGTTTGCCCTGATCTGCATCGCTGTGG CCGAGGCCAAGCCCACCGAGAACAACGAAGACTTCAACATCGTGGCCGTGGCCAGCAACTTCGCG ACCACGGATCTCGATGCTGACCGCGGGAAGTTGCCCGGCAAGAAGCTGCCGCTGGAGGTGCTCAA AGAGTTGGAAGCCAATGCCCGGAAAGCTGGCTGCACCAGGGGCTGTCTGATCTGCCTGTCCCACAT CAAGTGCACGCCCAAGATGAAGAAGTTCATCCCAGGACGCTGCCACACCTACGAAGGCGACAAAG AGTCCGCACAGGGCGGCATAGGCGAGGCGATCGTCGACATTCCTGAGATTCCTGGGTTCAAGGAC TTGGAGCCCTTGGAGCAGTTCATCGCACAGGTCGATCTGTGTGTGGACTGCACAACTGGCTGCCTC AAAGGGCTTGCCAACGTGCAGTGTTCTGACCTGCTCAAGAAGTGGCTGCCGCAACGCTGTGCGACC TTTGCCAGCAAGATCCAGGGCCAGGTGGACAAGATCAAGGGGGCCGGTGGTGACTAAGGATCCTA GGGCCGCAGCTCGCTTTCTTGCTGTCCAATTTCTATTAAAGGTTCCTTTGTTCCCTAAGTCCAACTA CTAAACTGGGGGATATTATGAAGGGCCTTGAGCATTTGGATTCTGCCTAATAAAAAACATTTATTT TCATTGCGTTTAGCTCGCTTTCTTGCTGTCCAATTTCTATTAAAGGTTCCTTTGTTCCCTAAGTCCAA CTACTAAACTGGGGGATATTATGAAGGGCCTTGAGCATTTGGATTCTGCCTAATAAAAAACATTTA TTTTCATTGCATTTAAATGTTTAAACAAAAAAAAAAAAAAAAAAAAAAAAAAAAAAAAAAGCAG GTGGGAGGGTACGTTCGTGATAAACAAACGGCCGCT |

### Examples

The following examples are provided solely to demonstrate the invention and to explain its aspects. The following examples should not be considered as limiting or demonstrating the full scope of the invention.

### Synthesis of EDA-PEGs-AG:

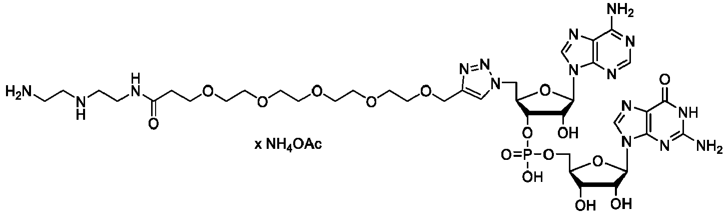

Diethylenetriamine (33.7 µl, 310 µmol, Sigma) was added to a solution of Alkyne-PEG5-NHS ester (12.5 mg, 31.2 µmol, Sigma) in DMF (200 µl). After 150 min of vortexing at 20°C aqueous solution of ammonium acetate (50 µl, 500 mM, HPLC-grade) and acetic acid (60 µl, 50 %) were added to afford a mixture of pH ~7. Next, solution of N₃-AG (28 µmol, 2.00 ml)³¹ and solution of Cu(II)-THPTA complex (60 µl, 50 mM) were combined with the mixture. After that, sodium ascorbate (31 mg, 157 µmol) was added, and the reaction mixture was stirred at 20°C and monitored by HPLC. After two hours solution of triethylammonium acetate (200 µl, 1 M, HPLC-grade), solution of Cu(II)-THPTA complex (20 µl, 50 mM), and sodium ascorbate (114 mg, 586 µmol) were added, and the reaction was continued overnight. The reaction was quenched with EDTA and resolved using HPLC - column: Gemini 5 µm NX-C18 110 Å 250 × 10 mm; flow 5 ml/min at 23°C; solvent A: 50 mM NH₄OAc pH 5.9; solvent B: acetonitrile; elution: from 0% to 41% B in 25 min, then from 41% to 100% B in 10 min, then 100% B for 5 min. HPLC eluate containing the desired product was freeze-dried and resuspended in water in tree repetitions to remove the traces of solvents and ammonium acetate. The final product EDA-PEG₅-AG was obtained in a form of solution of an ion pair with ammonium acetate (9.2 µmol, 95% purity by HPLC) with 33% yield. MS ESI(-): m/z = 1025.8, calculated for C₃₈H₅₈N₁₆O₁₆P⁻ m/z: 1025.4.

### Synthesis of N₃-GDP

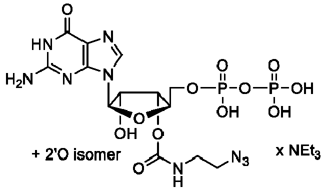

1,1-Carbonyldiimidazole (CDI) (1.21 g, 7.5 mmol, 10 eqiuv.) was added to a solution of GDP (9052.5 mOD₂₆₀, 500 mg, 750 µmol) in DMSO (15 mL). The reaction mixture was stirred at room temperature and monitor by RP-HPLC. After five hours reaction, CDI excess was hydrolysed with water (200 µL), and the 2-azidoethylamine (7.5 mmol, 620 µL, 10 equiv.) was added, followed by DBU (3.0 mmol, 448 µL). Resulting mixture was stirred overnight at room temperature. The reaction progress was monitored by RP-HPLC. After completion of linker attachment, the solution wad was diluted with hydrochloric acid (1% aq.) until the pH 1 was reached, and the mixture was stirred overnight at room temp. The neutralised mixture was purified on DEAE-Sephadex column. The eluate was concentrated to yield a triethylammonium salt of final product as a mixture of 2'-*O*/3'*O* regioisomers (7638 mOD₂₆₀, 633 µmol, 84%).

### Synthesis of N₃-M⁷GDP

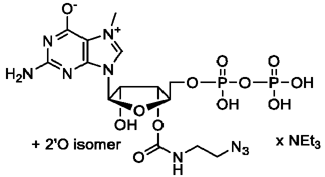

Methyl iodide (6.33 mmol, 394 µL, 10 equiv.) was added to a suspension of N₃-GDP (mixture of isomers) (7638 mOD₂₆₀, 350 mg, 633 µmol) in DMSO (6.33 mL), and resulting mixture was stirred at room temperature. The reaction progress was monitored by RP-HPLC. After four hours the reaction was quenched with water (60 mL) and extracted with diethyl ether (2x 50 mL). The aqueous phase was neutralised with NaHCO₃ and purified on a DEAE-Sephadex column. The eluate was concentrated to yield a triethylammonium salt of final product as a mixture of 2'-*O*/3'*O* regioisomers (5282.6 mOD₂₆₀, 463 µmol, 82%).

### Synthesis of N₃-m⁷GpppAₘG (or N₃-Cap1)

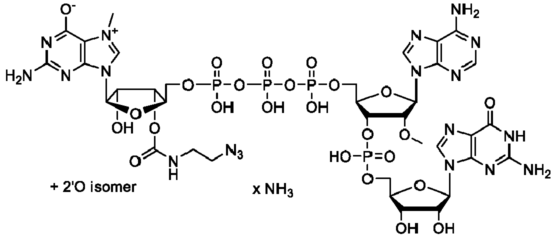

N₃-m⁷GDP (mix of regioisomers) (1778 mOD₂₆₀, 156 µmol, 88 mg, 1.5 equiv) and Im-pAₘpG (2833.6 mOD₂₆₀, 78.5 mg, 104 µmol)²¹ were suspended in DMF (2.0 mL) and ZnCl₂ (228 mg, 1.67 mmol, 16 equiv.) was added. Resulting mixture was stirred, and the reaction progress was monitored by RP-HPLC. After completion, reaction was terminated by addition of aqueous EDTA (20 mL), and the pH was adjusted to 6 with solid NaHCO₃. The product was purified by semipreparative RP-HPLC with use of linear gradient (0-40%) of acetonitrile in ammonium acetate (pH 5.9, 0.05 M) over 60 min. Collected fractions were combined and concentrated to remove the excess of ACN. The product (mixture of 2'-O/3'O regioisomers) (3232 mOD₂₆₀, 55 µmol, 53%) was obtained as a white powder after lyophilisation. ¹H NMR (500 MHz, D₂O, 25°C): δ = 9.21 (s, 1H, H8_{m7G}, isomer 3'), 9.17 (s, 1H, H8_{m7G}, isomer 2') 8.57 (s, 2H, H8_{A}, isomer 2' and 3'), 8.31 (s, 2H, H2_{A}, isomer 2' and 3'), 8.04 (s, 2H, H8_{G}, isomer 2' and 3'), 6.10 (m, 2H, H1'_{A}, isomer 2' and 3'), 6.02 (d, ³*J*_{H-H} = 3.3 Hz, 1H, H1'_{m7G} isomer 2'), 5.89 (d, ³*J*_{H-H} = 5.6 Hz, 1H, H1'_{m7G} isomer 3'), 5.80 (m, 2H, H1'_{G}, isomer 2' and 3'), 5.47 (m, 1H, H2'_{m7G} isomer 2'), 5.23 (m, 1H, H3'_{m7G} isomer 3'), 4.93 (m, 2H, H3'_{A}, isomer 2' and 3'), 4.78 (m, overlapped with HDO, 2H, H2'_{G}, H2'_{m7G} isomer 3'), 4.67 (m, 1H, H3'_{m7G} isomer 2'), 4.54-4.46 (m, 2H, H3'_{G}, H4'_{m7G} isomer 3', 2H, H4'_{A}, isomer 2' and 3'), 4.45-4.41 (m, 2H, H2'_{A}, isomer 2' and 3'), 4.35 (m, 2H, H4'_{G}, H4'_{m7G} isomer 2', 4H, H5'_{m7G}, H5"_{m7G}, isomer 2' and 3'), 4.30-4.14 (m, 8H, H5'_{G}, H5"_{G}, H5'_{A}, H5"A, isomer 2' and 3'), 4.04 (s, 6H *N*7-CH_{3 m7G}, isomer 2' and 3'), 3.49 (m, 6H, CH₃, 2'-O-CH₃, isomer 2' and 3'), 3.45-3.30 (m, 8H, CH₂-CH₂, isomer 2' and 3').

### Synthesis of EDA-PEGs-m⁷GpppAₘG (or EDA-PEGs-Capl)

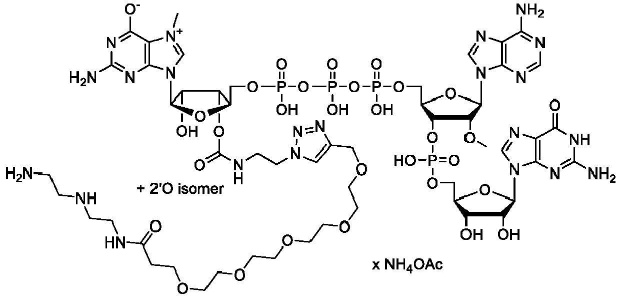

Diethylenetriamine (9.6 µl, 90 µmol, Sigma) was added to a solution of Alkyne-PEG5-NHS ester (12.0 mg, 29.9 µmol, Sigma) in DMF (180 µl). After 60 min of vortexing at 25°C aqueous solution of triethylammonium acetate (520 µl, 50 mM, HPLC-grade) and acetic acid (62 µl, 50 %) were added to afford a mixture of pH 6-7. Next, solution of N₃-m⁷GpppAₘG (400 µl, 26 µmol) and solution of Cu(II)-THPTA complex (96 µl, 50 mM) were combined with the mixture. After that, sodium ascorbate (49 mg, 248 µmol) was added, and the reaction mixture was stirred at 25°C and monitored by HPLC. After two hours solution of Cu(II)-THPTA complex (48 µl, 50 mM), and sodium ascorbate (25 mg, 126 µmol) were added, and the reaction was continued overnight. The reaction was quenched with EDTA and resolved using HPLC - column: Gemini 5 µm NX-C18 110 Å 250 × 10 mm; flow 5 ml/min at 23°C; solvent A: 50 mM NH₄OAc pH 5.9; solvent B: water/acetonitrile mixture 1:2 (v/v); elution: from 0% to 25% B in 45 min, then from 25% to 100% B in 5 min, then 100% B for 5 min. HPLC eluate containing the desired product was freeze-dried and resuspended in water in tree repetitions to remove the traces of solvents and ammonium acetate. The final product EDA-PEGs-AG was obtained in a form of solution of an ion pair with ammonium acetate (10.5 µmol, 99% purity by HPLC) with 40% yield. HRMS: mz/ = 1631.42458, 815.20983 (Teor. [M-H]⁻: m/z = 1631.42375, [M-2H]²⁻: m/z = 815.20824)

### DNA template preparation

The plasmids were prepared by cloning a sequence of choice (Table 4), containing a T7 RNA polymerase promoter and a site for a specific restrictase cleavage, into a pJet vector. To afford the desired DNA template for IVT the plasmids were linearised with a restriction enzyme according to manufacturer's protocol (Thermo). For details see Table 2.

**General protocol for preparation of EDA-linker-RNA (pre-circRNA 2, 4-6, 8, 10, 12, 13, 15, 17)**

| | C_{stock} | V_{stock} [µl] | C_{fin} |
|---|---|---|---|
| H₂O | | up to 50 µl | |
| Transcription Buffer x5 | x5 | 10 | x1 |
| U/A/CTP [mM] | 33.3 | 9 | 6 |
| GTP [mM] | 100 | 2 | 4 |
| EDA-linker-AG (or EDA-linker-Cap) [mM] | 40 | 15 | 12 |
| DNA template [ng/µl] | 400 | 5 | 40 |
| MgCl₂ [mM] | 1 000 | 1.25 | 25 |
| Ppase [U/µl] | 0.1 | 1 | 0.002 |
| Ribolock [U/µl] | 40 | 1.25 | 1 |
| T7 RNAP HC [U/µl] | 200 | 5 | 20 |

All ingredients of the reaction mixture listed in the table above were mixed in room temperature in the following order: water, Transcription Buffer x5 (200 mM Tris-HCl pH 7.9, 30 mM MgCl₂, 50 mM DTT, 50 mM NaCl and 10 mM spermidine, Thermo), NTPs (Thermo), EDA-linker-AG (or EDA-linker-Cap), DNA template, MgCl₂, Ribolock (Thermo), and T7 RNA polymerase HC (Thermo). The reaction mixture was vortexed, gently centrifuged, and incubated for 2 h at 37°C. Next, DNase I (2.0 µl, 1 U/µl, Thermo) was added, and the mixture was incubated for 30 min at 37°C. The RNA product was isolated form reaction mixture using Monarch RNA cleanup kit (500 µg, NEB). The isolated RNA (-200 µg) was resolved using RP-HPLC - column: PolymerX RP-1 3 µm 100 Å, 4.1 × 150 mm; flow 1.0 ml/min at 60°C; solvent A: 100 mM TEAA pH 7.0; solvent B: 200 mM TEAA pH 7.0 / acetonitrile 1:1 (vol/vol); elution: from 20% to 32.5% B in 25 min, then from 32.5% to 100% B in 2 min, then 100% B for 2 min. HPLC eluate containing the desired RNA product was concentrated by precipitation: sodium acetate (0.1 V, 3.0 M, pH 5.2), and isopropanol (1.0-1.5 V) were added to the eluate (initial volume = 1 V), and the solution was cooled for 30 min at -80°C or overnight at -20°C. The pellet was centrifuged (30 min, 14 000 g, 4°C), washed with 80 % ethanol (1.0 ml), centrifuged (10 min, 14 000 g, 4°C), dried *in vacuo,* and suspended in water to afford the RNA product (100-180 µg, 50-90% of the crude product).

### General protocol for preparation of p-RNA (pre-circRNA 1, 3, 7, 9, 11, 14, 16)

| | C_{stock} | V_{stock} [µl] | C_{fin} |
|---|---|---|---|
| H₂O | | up to 50 µl | |
| Transcription Buffer x5 | x5 | 10 | x1 |
| U/A/CTP [mM] | 33.3 | 9 | 6 |
| GTP [mM] | 100 | 1.5 | 3 |
| GMP [mM] | 100 | 10 | 20 |
| DNA template [ng/µl] | 400 | 5 | 40 |
| MgCl₂ [mM] | 1 000 | 1.25 | 25 |
| Ppase [U/µl] | 0.1 | 1 | 0.002 |
| Ribolock [U/µl] | 40 | 1.25 | 1 |
| T7 RNAP HC [U/µl] | 200 | 5 | 20 |

All ingredients of the reaction mixture listed in the table above were mixed at 37 °C in the following order: water, Transcription Buffer x5 (200 mM Tris-HCl pH 7.9, 30 mM MgCl₂, 50 mM DTT, 50 mM NaCl and 10 mM spermidine, Thermo), NTPs (Thermo), GMP (preincubated at 37°C), DNA template, MgCl₂, Ribolock (Thermo), and T7 RNA polymerase HC (Thermo). The reaction mixture was vortexed, gently centrifuged, and incubated for 2 h at 37°C. Next, DNase I (2.0 µl, 1 U/µl, Thermo) was added, and the mixture was incubated for 30 min at 37°C. The crude RNA product was isolated form reaction mixture using Monarch RNA cleanup kit (500 µg, NEB). The isolated crude RNA (-150 µg, 2.5-3.5 g of RNA per one litter of IVT reaction mixture) was resolved using RP-HPLC - column: PolymerX RP-1 3 µm 100 Å, 4.1 × 150 mm; flow 1.0 ml/min at 60°C; solvent A: 100 mM TEAA pH 7.0; solvent B: 200 mM TEAA pH 7.0 / acetonitrile 1: 1 (vol/vol); elution: from 20% to 32.5% B in 25 min, then from 32.5% to 100% B in 2 min, then 100% B for 2 min. HPLC eluate containing the desired RNA product was concentrated by precipitation: sodium acetate (0.1 V, 3.0 M, pH 5.2), and isopropanol (1.0-1.5 V) were added to the eluate (initial volume = 1 V), and the solution was cooled for 30 min at -80°C or overnight at -20°C. The pellet was centrifuged (30 min, 14 000 g, 4°C), washed with 80 % ethanol (1.0 ml), centrifuged (10 min, 14 000 g, 4°C), dried *in vacuo,* and suspended in water to afford the RNA product (70-130 µg, 50-90% of the crude product).

### Chemical circularization

A diluted solution of the precursor RNA (30-40 µg in 490 µl, ~140 µM, pre-circRNA 2, 4-6, 8, 10, 12, 13, 15, 17) was mixed with buffer (70 µl, 800 mM KH₂PO₄, 200 mM NaCl, pH 7.0) and incubated at 65°C for 5 min. After that, hot solution was transferred on bench, where it cooled to room temperature. After 10-15 min at room temperature, fresh solution of sodium periodate (70 µl, 10 mM) was added, and the reaction mixture was immediately placed in dark and incubated at 25°C. After 30 min, fresh solution of sodium cyanoborohydride (70 µl, 200 mM) was added, and the incubation was continued for next 90 min. The crude circRNA product was isolated form reaction mixture using Monarch RNA cleanup kit (50 µg, NEB). The isolated RNA (80-99% of the initial amount) was resolved using RP-HPLC - column: PolymerX RP-1 3 µm 100 Å, 4.1 × 150 mm; flow 1.0 ml/min at 60°C; solvent A: 100 mM HAA pH 7.0; solvent B: 100 mM HAA pH 7 in 75 % acetonitrile; elution: from 55% to 60% B in 25 min, then from 60% to 100% B in 2 min, then 100% B for 2 min. HPLC eluate containing the desired RNA product was concentrated by precipitation: sodium acetate (0.1 V, 3.0 M, pH 5.2), and isopropanol (1.0-1.5 V) were added to the eluate (initial volume = 1 V), and the solution was cooled for 30 min at - 80°C or overnight at -20°C. The pellet was centrifuged (30 min, 14 000 g, 4°C), washed with 80 % ethanol (1.0 ml), centrifuged (10 min, 14 000 g, 4°C), dried *in vacuo,* and suspended in water to afford the circRNA product (5-10 µg, 15-25% of the initial amount). For additional purification and removal of linear RNA, the product was treated with RNase R as described below (see section RNase R digestion).

### Circularization with complementary oligonucleotide

A diluted solution of the precursor RNA (30-40 µg in 480 µl, ~145 µM, pre-circRNA 2, 4-6, 8, 10, 12, 13, 15, 17) was mixed with buffer (70 µl, 800 mM KH₂PO₄, 200 mM NaCl, pH 7.0), and complementary oligonucleotide (-11 µl, 10 µM, 1.5 eq, see Table 3 for sequence) and incubated at 65°C for 5 min. After that, hot solution was transferred on bench, where it cooled to room temperature. After 10-15 min at room temperature, fresh solution of sodium periodate (70 µl, 10 mM) was added, and the reaction mixture was immediately placed in dark and incubated at 25°C. After 30 min, fresh solution of sodium cyanoborohydride (70 µl, 200 mM) was added, and the incubation was continued for next 90 min. The crude circRNA product was isolated form reaction mixture using Monarch RNA cleanup kit (50 µg, NEB). The isolated RNA was resolved using RP-HPLC - column: PolymerX RP-1 3 µm 100 Å, 4.1 × 150 mm; flow 1.0 ml/min at 60°C; solvent A: 100 mM HAA pH 7.0; solvent B: 100 mM HAA pH 7 in 75 % acetonitrile; elution: from 55% to 60% B in 25 min, then from 60% to 100% B in 2 min, then 100% B for 2 min. HPLC eluate containing the desired RNA product was concentrated by precipitation: sodium acetate (0.1 V, 3.0 M, pH 5.2), and isopropanol (1.0-1.5 V) were added to the eluate (initial volume = 1 V), and the solution was cooled for 30 min at -80°C or overnight at -20°C. The pellet was centrifuged (30 min, 14 000 g, 4°C), washed with 80 % ethanol (1.0 ml), centrifuged (10 min, 14 000 g, 4°C), dried *in vacuo,* and suspended in water to afford the circRNA product (5-10 µg, 15-25% of the initial amount). For additional purification and removal of linear RNA, the product was treated with RNase R as described below (see section RNase R digestion).

### Enzymatic circularization with T4 RNA Ligase I

A diluted solution of the precursor RNA (10-15 µg in 95 µl, -150 µM, pre-circRNA 1, 3, 7, 9, 11, 14, 16) was mixed with buffer (5 µl, 100 mM Tris-HCl, 500 mM NaCl, pH 7.0) and incubated at 65°C for 5 min. After that, hot solution was transferred on bench, where it cooled to room temperature. After 10-15 min at room temperature, T4 RNA ligase 1 buffer x10 (15 µl, 500 mM Tris-HCl, 100 mM MgCl₂, 10 mM DTT, pH 7.5, NEB), PEG 8000 (15 µl, 50%, NEB), ATP (0.75 µl, 10 mM, NEB), RiboLock RNase inhibitor (3.75 µl, 40 U/µl, Thermo), and T4 RNA ligase 1 (15 µl, 10 U/µl, NEB) were added, and the reaction mixture was incubated at 25°C for 150 min. The crude circRNA product was isolated form reaction mixture using Monarch RNA cleanup kit (10 µg, NEB). The isolated RNA (80-99% of the initial amount) was resolved using RP-HPLC - column: RNASept Prep 2 µm 100 Å, 7.8 × 50 mm; flow 1.0 ml/min at 60°C; solvent A: 100 mM HAA pH 7.0 in 10% acetonitrile; solvent B: 100 mM HAA pH 7 in 75 % acetonitrile; elution: from 20% to 70% B in 25 min, then from 70% to 100% B in 2 min, then 100% B for 2 min. HPLC eluate containing the desired RNA product was concentrated by precipitation: sodium acetate (0.1 V, 3.0 M, pH 5.2), and isopropanol (1.0-1.5 V) were added to the eluate (initial volume = 1 V), and the solution was cooled for 30 min at -80°C or overnight at -20°C. The pellet was centrifuged (30 min, 14 000 g, 4°C), washed with 80 % ethanol (1.0 ml), centrifuged (10 min, 14 000 g, 4°C), dried *in vacuo,* and suspended in water to afford the circRNA product (1-4 µg, 10-30% of the crude product). For additional purification and removal of linear RNA, the product was treated with RNase R as described below (see section RNase R digestion).

### Enzymatic circularization with T4 RNA Ligase II

A diluted solution of the precursor RNA (30-40 µg in 220 µl, -320 µM, pre-circRNA 1, 3, 7, 9, 11, 14, 16) was mixed with complementary oligonucleotide (~11 µl, 10 µM, 1.5 eq, see Table 3 for sequence) and incubated at 65°C for 5 min. After that, hot solution was transferred on bench, where it cooled to room temperature. After 10-15 min at room temperature, T4 RNA ligase 2 buffer x10 (35 µl, 500 mM Tris-HCl, 20 mM MgCl₂, 10 mM DTT, 4 mM ATP, pH 7.5, NEB), PEG 8000 (70 µl, 50%, NEB), RiboLock RNase inhibitor (8.75 µl, 40 U/µl, Thermo), and T4 RNA ligase 2 (3.5 µl, 10 U/µl, NEB) were added, and the reaction mixture was incubated at 25°C for 150 min. The crude circRNA product was isolated form reaction mixture using Monarch RNA cleanup kit (500 µg, NEB). The isolated RNA (80-99% of the initial amount) was resolved using RP-HPLC - column PolymerX RP-1 3 µm 100 Å, 4.1 × 150 mm; flow 1.0 ml/min at 60°C; solvent A: 100 mM HAA pH 7.0; solvent B: 100 mM HAA pH 7 in 75 % acetonitrile; elution: from 55% to 60% B in 25 min, then from 60% to 100% B in 2 min, then 100% B for 2 min. HPLC eluate containing the desired RNA product was concentrated by precipitation: sodium acetate (0.1 V, 3.0 M, pH 5.2), and isopropanol (1.0-1.5 V) were added to the eluate (initial volume = 1 V), and the solution was cooled for 30 min at -80°C or overnight at -20°C. The pellet was centrifuged (30 min, 14 000 g, 4°C), washed with 80 % ethanol (1.0 ml), centrifuged (10 min, 14 000 g, 4°C), dried *in vacuo,* and suspended in water to afford the circRNA product (5-10 µg, 15-25% of the initial amount). For additional purification and removal of linear RNA, the product was treated with RNase R as described below (see section RNase R digestion).

### RP-HPLC of circRNA

All HPLC separations were performed on modular, low-pressure gradient HPLC apparatus manufactured by Shimadzu (LC-40/LC-20 series) with thermostated column holder or column oven, DAD detector and fluorescence detector. Mobile phase was composed of non-autoclaved MQ water, HPLC-grade acetonitrile (J.T.Baker), high quality triethylamine (Bioultra, Sigma), HPLC-grade n-hexylamine (Ottokemi), and HPLC-grade glacial acetic acid (J.T.Baker). Solvents of desired composition, concentration and pH were filtered with 0.45 µm filter and stored in dark at 4°C for longer periods of time. Chromatograms were recorded with detection of absorbance at 260 nm (ref 360 nm, 10 nm bandwidth), absorbance spectrum (220-700 nm), and fluorescence of interest (Cy3 - 550/565, Cy5-650/665).

### RNase R digestion

A solution of RNA (43 µl, 10 µg) was incubated at 65°C for 5 min and rapidly cooled in ice bath. After 5 min at 0-4°C, RNase R buffer x10 (5 µl, 200 mM Tris-HCl, 1 M KCl, 1 mM MgCl₂, pH 7.5, ABM), RiboLock RNase inhibitor (1.25 µl, 40 U/µl, Thermo), and RNase R (1 µl, 10 U/µl, ABM) were added. After 30 min of incubation at 37°C the products of digestion were analyzed with PAGE directly or isolated form reaction mixture using Monarch RNA cleanup kit (10 µg, NEB).

### RNase H assay

A solution (8.5 µl) containing circRNA2 (100 ng), DNA oligonucleotide (0 or 150 nM, CCTTCAGCTCGATGCGGTTCA), and RNase H buffer x1 (20 mM Tris-HCl, 10 mM KCl, 8 mM MgCl₂, 1 mM DTT, Thermo) was heated to 65°C for 5 min and gradually cooled to 37°C for 30 min. Next, RiboLock RNase inhibitor (0.5 µl, 40 U/µl, Thermo), and RNase H (1 µl, 5 U/µl, Thermo) were added. After 30 min of incubation at 37°C the products of digestion were directly analyzed with PAGE.

### FRET probe synthesis

The Cy5-RNA1-Cy3 FRET probe was synthesised according to previously reported protocol.³¹ First in vitro transcription of RNA1 sequence was performed in presence of N₃-AG transcription initiator. The crude product of IVT was isolated by Monarch RNA cleanup kit (NEB) and subjected to 5'-Cy5 and 3'-Cy3 dual labeling. The crude labeling product was isolated from labeling reaction mixture by Monarch RNA cleanup kit (NEB) and subsequently resolved with HPLC. The HPLC eluate containing the desired product was concentrated by precipitation dried *in vacuo* and suspended in water to afford the Cy5-RNA1-Cy3 FRET probe solution.

### FRET experiments

The Cy5-RNA1-Cy3 FRET probe solution was diluted with buffer (80 mM KH₂PO₄, 20 mM NaCl, pH 7.0, final volume of 100 µl) to afford final probe concentration of 100 nM and transferred to a quartz fluorescence cuvette (1 × 1 × 350 mm). Optionally, solution of a complementary DNA oligonucleotide (2 µl, 10 µM, ON2-ON5) was added. The mixture was heated to from 20 to 90 °C in 8 min and gradually cooled to 20°C or 4°C in 20-30 min. Emission spectra were recorded on a Cary Eclipse spectrofluorometer (Agilent), equipped with a xenon lamp, single cell peltier accessory (spvf 1x0, Agilent), excitation at 500 nm, emission 510-800 nm, 10 mm slit.

### Cell culture and transfections

Analysis was performed in four cell lines, i.e. HEK 293T, Hep G2, A549, and HeLa, as well as in mouse dendritic cells and macrophages established from bone marrow monocytes. One day before transfection, 8×10^3 cells were seeded per well of a 96-well plate. The next day, cells were transfected with RNA. In each cell line, each RNA variant was transfected in triplicate. Transfection was performed with Lipofectamine Messenger MAX (Invitrogen, Waltham, MA, USA), according to the manufacturer's instructions. During transfection, the molar concentration of each RNA was taken into account so that an equal amount of RNA molecules was added to each well. Briefly, for each reaction 0,15 µL of Lipofectamine was first diluted (Mix 1) in 5 µL of Opti-MEM (GIBCO, Grand Island, NY, USA) medium and incubated at room temperature for 10 minutes. In another tube, the RNA was diluted in 5 µL of Opti-MEM medium (Mix 2). Next, both Mix 1 and Mix 2 were mixed and incubated for 5 minutes at room temperature. Afterward, 10 µL of the mixture was transferred to the appropriate well of a 96-well plate containing previously seeded cells. After the transfection, the cells were incubated in standard culture conditions (37°C, 5% CO2, saturating humidity). At appropriate time points, 20 uL of medium from each well was withdrawn to test the amount of produced protein, and the remaining medium was completely replaced with the fresh one. The amount of luciferase released into the medium was analyzed using the GLuc GLOW Assay (NanoLight Technologies, Norman, OK, USA) reagents, according to the manufacturer's instructions. Luminescence was read in white, opaque plates. Measurements were made using an EnVision plate reader (Perkin Elmer, Waltham, MA, USA).

### References

(1) Qin, S.; Tang, X.; Chen, Y.; Chen, K.; Fan, N.; Xiao, W.; Zheng, Q.; Li, G.; Teng, Y.; Wu, M.; et al. mRNA-based therapeutics: powerful and versatile tools to combat diseases. Signal Transduction and Targeted Therapy 2022, 7 (1). DOI: 10.1038/s41392-022-01007-w (acccessed 2022-10-07T12:03:06). Janowski, M.; Andrzejewska, A. The legacy of mRNA engineering: A lineup of pioneers for the Nobel Prize. Molecular Therapy - Nucleic Acids 2022, 29, 272-284. DOI: 10.1016/j.omtn.2022.07.003 (acccessed 2022-10-07T11:43:22).
(2) Garneau, N. L.; Wilusz, J.; Wilusz, C. J. The highways and byways of mRNA decay. Nature Reviews Molecular Cell Biology 2007, 8 (2), 113-126. DOI: 10.1038/nrm2104. Houseley, J.; Tollervey, D. The Many Pathways of RNA Degradation. Cell 2009, 136 (4), 763-776. DOI: 10.1016/j.cell.2009.01.019 (acccessed 2022-10-07T12:34:14).
(3) Jeck, W. R.; Sharpless, N. E. Detecting and characterizing circular RNAs. Nature Biotechnology 2014, 32 (5), 453-461. DOI: 10.1038/nbt.2890 (acccessed 2022-10-07T12:45:35).
(4) Dolinnaya, N. G.; Blumenfeld, M.; Merenkova, I. N.; Oretskaya, T. S.; Krynetskaya, N.; Ivanovskaya, M. G.; Vasseur, M.; Shabarova, Z. A. Oligonucleotide circularization by template-directed chemical ligation. Nucleic Acids Research 1993, 21 (23), 5403-5407. DOI: 10.1093/nar/21.23.5403 (acccessed 10/7/2022). Fedorova, O. A.; Gottikh, M. B.; Oretskaya, T. S.; Shabarova, Z. A. Cyanogen Bromide-Induced Chemical Ligation: Mechanism and Optimization of the Reaction Conditions. Nucleosides and Nucleotides 1996, 15 (6), 1137-1147. DOI: 10.1080/07328319608007382.
(5) Nakamoto, K.; Abe, N.; Tsuji, G.; Kimura, Y.; Tomoike, F.; Shimizu, Y.; Abe, H. Chemically synthesized circular RNAs with phosphoramidate linkages enable rolling circle translation. Chemical Communications 2020, 56 (46), 6217-6220, 10.1039/D0CC02140G. DOI: 10.1039/D0CC02140G.
(6) Rigden, J. E.; Rezaian, M. A. In Vitro synthesis of an infectious viroid: Analysis of the infectivity of monomeric linear CEV. Virology 1992, 186 (1), 201-206. DOI: https://doi.org/10.1016/0042-6822(92)90074-Y.
(7) Chen, C.-y.; Sarnow, P. Initiation of Protein Synthesis by the Eukaryotic Translational Apparatus on Circular RNAs. Science 1995, 268 (5209), 415-417. DOI: 10.1126/science.7536344 (acccessed 2022/10/07).
(8) Litke, J. L.; Jaffrey, S. R. Highly efficient expression of circular RNA aptamers in cells using autocatalytic transcripts. Nature Biotechnology 2019, 37 (6), 667-675. DOI: 10.1038/s41587-019-0090-6 (acccessed 2022-10-07T13:55:58).
(9) Kumar, A.; Palmer, N.; Miyasaki, K.; Finburgh, E.; Xiang, Y.; Portell, A.; Dailamy, A.; Suhardjo, A.; Chew, W. L.; Kwon, E. J.; et al. Extensive <i>in vitro</i> and <i>in vivo</i> protein translation via <i>in situ</i> circularized RNAs. Cold Spring Harbor Laboratory: 2022.
(10) Ford, E.; Ares, M. Synthesis of circular RNA in bacteria and yeast using RNA cyclase ribozymes derived from a group I intron of phage T4. Proceedings of the National Academy of Sciences 1994, 91 (8), 3117-3121. DOI: 10.1073/pnas.91.8.3117 (acccessed 2022-10-07T13:11:48).
(11) Wesselhoeft, R. A.; Kowalski, P. S.; Anderson, D. G. Engineering circular RNA for potent and stable translation in eukaryotic cells. Nature Communications 2018, 9 (1). DOI: 10.1038/s41467-018-05096-6 (acccessed 2022-10-07T13:35:12).
(12) Wesselhoeft, R. A.; Kowalski, P. S.; Parker-Hale, F. C.; Huang, Y.; Bisaria, N.; Anderson, D. G. RNA Circularization Diminishes Immunogenicity and Can Extend Translation Duration In Vivo. Molecular Cell 2019, 74 (3), 508-520.e504. DOI: 10.1016/j.molcel.2019.02.015 (acccessed 2022-10-07T13:26:51).
(13) Ni, L.; Yamada, T.; Murata, A.; Nakatani, K. Mismatch binding ligand upregulated back-splicing reaction producing circular RNA in a cellular model. Chemical Communications 2022, 58 (22), 3629-3632. DOI: 10.1039/dlcc06936e (acccessed 2022-10-07T13:11:41).
(14) Qu, L.; Yi, Z.; Shen, Y.; Lin, L.; Chen, F.; Xu, Y.; Wu, Z.; Tang, H.; Zhang, X.; Tian, F.; et al. Circular RNA vaccines against SARS-CoV-2 and emerging variants. Cell 2022, 185 (10), 1728-1744.e1716. DOI: 10.1016/j.cell.2022.03.044 (acccessed 2022-10-07T13:26:16).
(15) Nichols, N. M.; Tabor, S.; McReynolds, L. A. RNA Ligases. Current Protocols in Molecular Biology 2008, 84 (1), 3.15.11-13.15.14, https://doi.org/10.1002/0471142727.mb0315s84. DOI: https://doi.org/10.1002/0471142727.mb0315s84 (acccessed 2022/10/07).
(16) Kaufmann, G.; Klein, T.; Littauer, U. Z. T4 RNA ligase: Substrate chain length requirements. FEBS Letters 1974, 46 (1-2), 271-275. DOI: 10.1016/0014-5793(74)80385-6 (acccessed 2022-10-07T13:11:45).
(17) Puttaraju, M.; Been, M. Group I permuted intron-exon (PIE) sequences self-splice to produce circular exons. Nucleic Acids Research 1992, 20 (20), 5357-5364. DOI: 10.1093/nar/20.20.5357 (acccessed 2022-10-10T16:55:04).
(18) Ramanathan, A.; Robb, G. B.; Chan, S.-H. mRNA capping: biological functions and applications. Nucleic Acids Research 2016, 44 (16), 7511-7526. DOI: 10.1093/nar/gkw551 (acccessed 10/7/2022). Topisirovic, I.; Svitkin Yuri, V.; Sonenberg, N.; Shatkin Aaron, J. Cap and cap-binding proteins in the control of gene expression. Wiley Interdisciplinary Reviews: RNA 2010, 2 (2), 277-298. DOI: 10.1002/wrna.52 (acccessed 2018/05/06). Mitchell, S. F.; Walker, S. E.; Algire, M. A.; Park, E.-H.; Hinnebusch, A. G.; Lorsch, J. R. The 5'-7-Methylguanosine Cap on Eukaryotic mRNAs Serves Both to Stimulate Canonical Translation Initiation and to Block an Alternative Pathway. Molecular Cell 2010, 39 (6), 950-962. DOI: 10.1016/j.molcel.2010.08.021 (acccessed 2022-10-07T14:25:08).
(19) Koch, A.; Aguilera, L.; Morisaki, T.; Munsky, B.; Stasevich, T. J. Quantifying the dynamics of IRES and cap translation with single-molecule resolution in live cells. Nature Structural & Molecular Biology 2020, 27 (12), 1095-1104. DOI: 10.1038/s41594-020-0504-7.
(20) Warminski, M.; Sikorski, P. J.; Kowalska, J.; Jemielity, J. Applications of Phosphate Modification and Labeling to Study (m)RNA Caps. Topics in Current Chemistry 2017, 375 (1), 16. DOI: 10.1007/s41061-017-0106-y.
(21) Sikorski, P. J.; Warminski, M.; Kubacka, D.; Ratajczak, T.; Nowis, D.; Kowalska, J.; Jemielity, J. The identity and methylation status of the first transcribed nucleotide in eukaryotic mRNA 5' cap modulates protein expression in living cells. Nucleic acids research 2020, 48 (4), 1607-1626.
(22) Chen, C.-K.; Cheng, R.; Demeter, J.; Chen, J.; Weingarten-Gabbay, S.; Jiang, L.; Snyder, M. P.; Weissman, J. S.; Segal, E.; Jackson, P. K.; et al. Structured elements drive extensive circular RNA translation. Molecular Cell 2021, 81 (20), 4300-4318.e4313. DOI: 10.1016/j.molcel.2021.07.042 (acccessed 2022/10/07). Yang, Y.; Fan, X.; Mao, M.; Song, X.; Wu, P.; Zhang, Y.; Jin, Y.; Yang, Y.; Chen, L.-L.; Wang, Y.; et al. Extensive translation of circular RNAs driven by N6-methyladenosine. Cell Research 2017, 27 (5), 626-641. DOI: 10.1038/cr.2017.31 (acccessed 2022-10-07T13:35:12). Fan, X.; Yang, Y.; Chen, C.; Wang, Z. Pervasive translation of circular RNAs driven by short IRES-like elements. Nature Communications 2022, 13 (1). DOI: 10.1038/s41467-022-31327-y (acccessed 2022-10-10T11:25:34).
(23) Lei, M.; Zheng, G.; Ning, Q.; Zheng, J.; Dong, D. Translation and functional roles of circular RNAs in human cancer. Molecular Cancer 2020, 19 (1). DOI: 10.1186/s12943-020-1135-7 (acccessed 2022-10-07T13:34:49). Prats, A.-C.; David, F.; Diallo, L. H.; Roussel, E.; Tatin, F.; Garmy-Susini, B.; Lacazette, E. Circular RNA, the Key for Translation. International Journal of Molecular Sciences 2020, 21 (22), 8591. DOI: 10.3390/ijms21228591 (acccessed 2022-10-07T13:30:21).
(24) Martinez-Salas, E.; Francisco-Velilla, R.; Fernandez-Chamorro, J.; Embarek, A. M. Insights into Structural and Mechanistic Features of Viral IRES Elements. Frontiers in Microbiology 2018, 8*,* Review. Marques, R.; Lacerda, R.; Romão, L. Internal Ribosome Entry Site (IRES)-Mediated Translation and Its Potential for Novel mRNA-Based Therapy Development. Biomedicines 2022, 10 (8), 1865. DOI: 10.3390/biomedicines10081865 (acccessed 2023-02-13T14:08:04). Chen, R.; Wang, S. K.; Belk, J. A.; Amaya, L.; Li, Z.; Cardenas, A.; Abe, B. T.; Chen, C.-K.; Wender, P. A.; Chang, H. Y. Engineering circular RNA for enhanced protein production. Nature Biotechnology 2022. DOI: 10.1038/s41587-022-01393-0 (acccessed 2022-10-07T11:58:17).
(25) Nowotny, M.; Gaidamakov, S. A.; Ghirlando, R.; Cerritelli, S. M.; Crouch, R. J.; Yang, W. Structure of human RNase H1 complexed with an RNA/DNA hybrid: insight into HIV reverse transcription. Molecular cell 2007, 28 (2), 264-276.
(26) Jacques, J.-P.; Susskind, M. M. Use of electrophoretic mobility to determine the secondary structure of a small antisense RNA. Nucleic Acids Research 1991, 19 (11), 2971-2977. DOI: 10.1093/nar/19.11.2971 (acccessed 2022-10-07T16:18:29). Cebrián, J.; Kadomatsu-Hermosa, M. J.; Castán, A.; Martinez, V.; Parra, C.; Fernández-Nestosa, M. J.; Schaerer, C.; Martinez-Robles, M.-L.; Hernández, P.; Krimer, D. B.; et al. Electrophoretic mobility of supercoiled, catenated and knotted DNA molecules. Nucleic Acids Research 2015, 43 (4), e24-e24. DOI: 10.1093/nar/gku1255 (acccessed 10/7/2022).
(27) Abe, B. T.; Wesselhoeft, R. A.; Chen, R.; Anderson, D. G.; Chang, H. Y. Circular RNA migration in agarose gel electrophoresis. Molecular Cell 2022, 82 (9), 1768-1777.e1763. DOI: 10.1016/j.molcel.2022.03.008 (acccessed 2022-10-07T13:26:14).
(28) Haque, F. M.; Grayson, S. M. The synthesis, properties and potential applications of cyclic polymers. Nature Chemistry 2020, 12 (5), 433-444. DOI: 10.1038/s41557-020-0440-5 (acccessed 2022-10-07T13:56:08).
(29) Vicens, Q.; Kieft, J. S. Thoughts on how to think (and talk) about RNA structure. Proceedings of the National Academy of Sciences 2022, 119 (17). DOI: 10.1073/pnas.2112677119 (acccessed 2022-10-07T12:03:32).
(30) Kibbe, W. A. OligoCalc: an online oligonucleotide properties calculator. Nucleic Acids Research 2007, 35 (suppl_2), W43-W46. DOI: 10.1093/nar/gkm234 (acccessed 10/10/2022).
(31) Mamot, A.; Sikorski, P. J.; Siekierska, A.; de Witte, P.; Kowalska, J.; Jemielity, J. Ethylenediamine derivatives efficiently react with oxidized RNA 3' ends providing access to mono and dually labelled RNA probes for enzymatic assays and in vivo translation. Nucleic Acids Research 2022, 50 (1), e3-e3. DOI: 10.1093/nar/gkab867 (acccessed 2/17/2022).

## Claims

1. A circRNA analog prepared by *in vitro* transcription reaction and a subsequent chemical circularization reaction wherein said circularization is obtained in the absence of protein enzymes or catalytic nucleic acid sequences.

2. A circRNA analog, that contains either a 7-methylguanosine cap analog structure or both a 7-methylguanosine cap analog structure and an internal ribosomal entry site sequence.

3. A circRNA analog according to formula 1 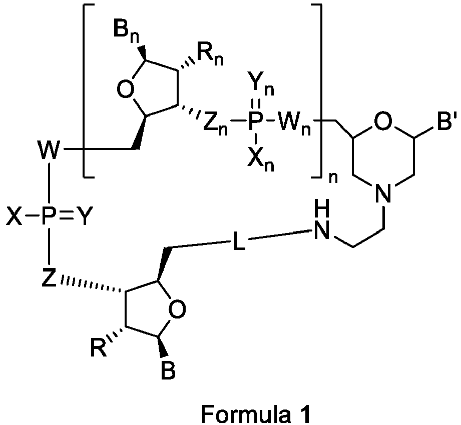 wherein:
n = 1-20 000;
X and each of X₁ through Xₙ is independently OH, BH₃, NH₂, or SH;
Y and each of Y₁ through Yₙ is independently O or S;
Z and each of Z₁ through Zₙ is independently O, S or NH;
Wand each of W₁ through Wₙ is independently O, S or NH;
R and each of R₁ through Rₙ is independently H, OH, alkyl, O-alkyl or halogen;
B, B' and each of B₁ through Bₙ is independently a natural, modified or unnatural nucleoside base;
L is a linear or branched linker.

4. A circRNA analog according to claim 3, wherein:
the linker L comprises of a natural, modified or unnatural 7-methylguanosine cap structure.

5. A circRNA analog according to claims 3-4, wherein:
the linker L comprises of a detectable label.

6. A circRNA analog according to formula 2 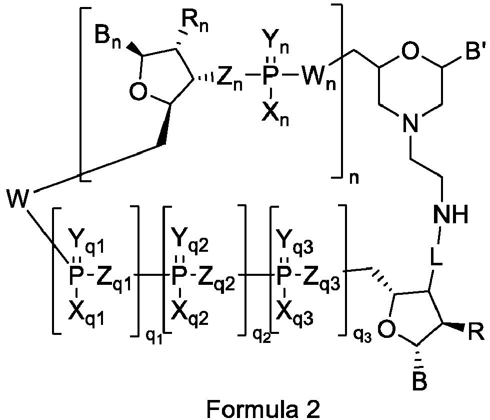 wherein:
n = 1-20 000;
each of q₁ through q₃ is independently 0 or 1;
X_{q1}, X_{q2}, X_{q3} and each of X₁ through Xₙ is independently OH, BH₃, NH₂, or SH;
Y_{q1}, Y_{q2}, Y_{q3} and each of Y₁ through Yₙ is independently O or S;
Z_{q1}, Z_{q2}, and Z_{q3} is independently O, S, NH, CH₂, C(halogen)₂ or CH(halogen);
each of Z₁ through Zₙ is independently O, S, or NH;
Wand each of W₁ through Wₙ is independently O, S or NH;
R and each of R₁ through Rₙ is independently H, OH, alkyl, O-alkyl or halogen;
B, B' and each of B₁ through Bₙ is independently a natural, modified or unnatural nucleoside base;
L is a linear or branched linker.

7. A circRNA analog according to formula 3 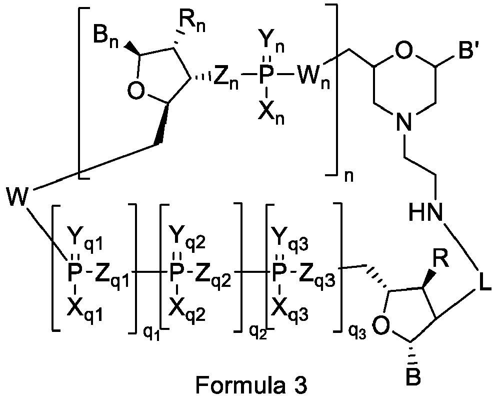 wherein:
n = 1-20 000;
each of q₁ through q₃ is independently 0 or 1;
X_{q1}, X_{q2}, X_{q3} and each of X₁ through Xₙ is independently OH, BH₃, NH₂, or SH;
Y_{q1}, Y_{q2}, Y_{q3} and each of Y₁ through Yₙ is independently O or S;
Z_{q1}, Z_{q2}, and Z_{q3} is independently O, S, NH, CH₂, C(halogen)₂ or CH(halogen);
each of Z₁ through Zₙ is independently O, S, or NH;
Wand each of W₁ through Wₙ is independently O, S or NH;
R and each of R₁ through Rₙ is independently H, OH, alkyl, O-alkyl or halogen;
B, B' and each of B₁ through Bₙ is independently a natural, modified or unnatural nucleoside base;
L is a linear or branched linker.

8. A circRNA analog according to formula 4 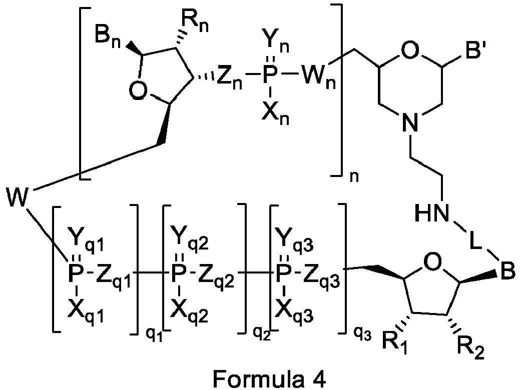 wherein:
n = 1-20 000;
each of q₁ through q₃ is independently 0 or 1;
X_{q1}, X_{q2}, X_{q3} and each of X₁ through Xₙ is independently OH, BH₃, NH₂, or SH;
Y_{q1}, Y_{q2}, Y_{q3} and each of Y₁ through Yₙ is independently O or S;
Z_{q1}, Z_{q2}, and Z_{q3} is independently O, S, NH, CH₂, C(halogen)₂ or CH(halogen);
each of Z₁ through Zₙ is independently O, S, or NH;
Wand each of W₁ through Wₙ is independently O, S or NH;
R and each of R₁ through Rₙ is independently H, OH, alkyl, O-alkyl or halogen;
B' and each of B₁ through Bₙ is independently a natural, modified or unnatural nucleoside base;
L is a linear or branched linker;
B is a modified or unnatural nucleoside base connected with the linker moiety L.

9. A circRNA analog according to formula 5 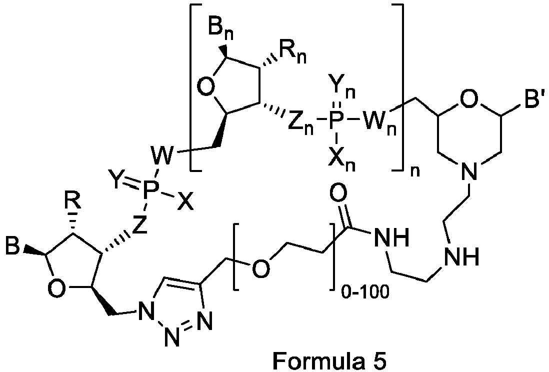 wherein:
n = 1-20 000
X and each of X₁ through Xₙ is independently OH, BH₃, NH₂, or SH;
Y and each of Y₁ through Yₙ is independently O or S;
Z and each of Z₁ through Zₙ is independently O, S or NH;
Wand each of W₁ through Wₙ is independently O, S or NH;
R and each of R₁ through Rₙ is independently H, OH, alkyl, O-alkyl or halogen;
B, B' and each of B₁ through Bₙ is independently a natural, modified or unnatural nucleoside base;

10. A circRNA analog according to formula 6 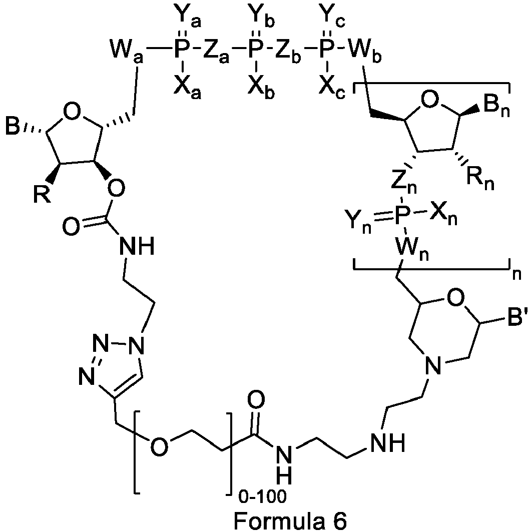 wherein:
n = 1-20 000;
Xₐ, X_{b}, X_{c} and each of X₁ through Xₙ is independently OH, BH₃, NH₂, or SH;
Yₐ, Y_{b}, Y_{c} and each of Y₁ through Yₙ is independently O or S;
each of Z₁ through Zₙ is independently O, S, or NH;
Xₐ and X_{b}, is independently O, S, NH, CH₂, C(halogen)₂ or CH(halogen);
Wₐ, W_{b} and each of W₁ through Wₙ is independently O, S or NH;
R each of R₁ through Rₙ is independently H, OH, alkyl, O-alkyl or halogen;
B, B' and each of B₁ through Bₙ is independently a natural, modified or unnatural nucleoside base;

11. A circRNA analog according to formula 6 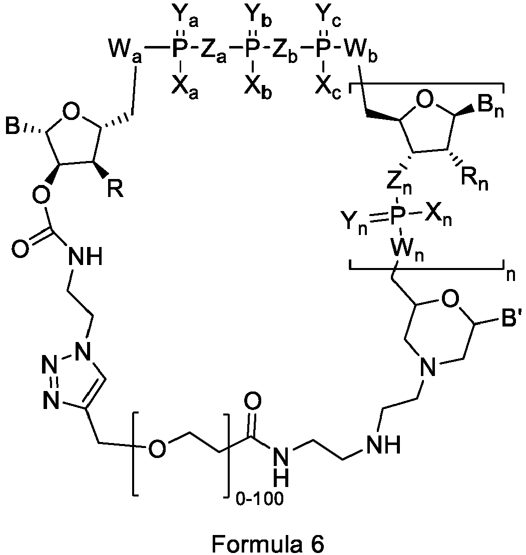 wherein:
n = 1-20 000;
Xₐ, X_{b}, X_{c} and each of X₁ through Xₙ is independently OH, BH₃, NH₂, or SH;
Yₐ, Y_{b}, Y_{c} and each of Y₁ through Yₙ is independently O or S;
each of Z₁ through Zₙ is independently O, S, or NH;
Xₐ and X_{b}, is independently O, S, NH, CH₂, C(halogen)₂ or CH(halogen);
Wₐ, W_{b} and each of W₁ through Wₙ is independently O, S or NH;
R each of R₁ through Rₙ is independently H, OH, alkyl, O-alkyl or halogen;
B, B' and each of B₁ through Bₙ is independently a natural, modified or unnatural nucleoside base;

12. A method of synthesis of circRNA analogs described in claims 3-11, **characterized by** that a circRNA analog precursor (formula 7) 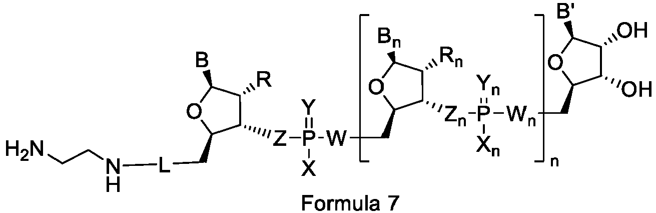 wherein:
n = 1-20 000;
X and each of X₁ through Xₙ is independently OH, BH₃, NH₂, or SH;
Y and each of Y₁ through Yₙ is independently O or S;
Z and each of Z₁ through Zₙ is independently O, S or NH;
Wand each of W₁ through Wₙ is independently O, S or NH;
R and each of R₁ through Rₙ is independently H, OH, alkyl, O-alkyl or halogen;
B, B' and each of B₁ through Bₙ is independently a natural, modified or unnatural nucleoside base;
L is a linear or branched linker;
is first oxidized during step (i) of the method, yielding a dialdehyde derivative (formula 8), 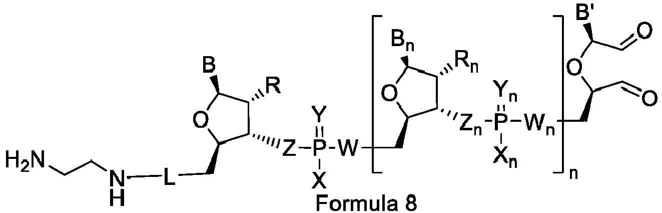 wherein:
n = 1-20 000;
X and each of X₁ through Xₙ is independently OH, BH₃, NH₂, or SH;
Y and each of Y₁ through Yₙ is independently O or S;
Z and each of Z₁ through Zₙ is independently O, S or NH;
Wand each of W₁ through Wₙ is independently O, S or NH;
R and each of R₁ through Rₙ is independently H, OH, alkyl, O-alkyl or halogen;
B, B' and each of B₁ through Bₙ is independently a natural, modified or unnatural nucleoside base;
L is a linear or branched linker;
which is next incubated in reductive environment during step (ii) of the method, yielding the desired circRNA analog.

13. A method, according to claim 12, **characterized by** that step (i) and step (ii) are carried out in one reactor.

14. A method, according to claim 12, **characterized by** that step (i) of the method is carried out in the presence of NaIO₄, preferably at a concentration below 10 mM, at a temperature below 60°C.

15. A method, according to claim 12, **characterized by** that step (ii) of the method is carried out in buffered solution, preferably at pH 5-8, in presence of NaBH₃CN below 100 mM.

16. A method, according to claim 12, **characterized by** that step (i) or step (ii) of the method are carried out in presence of a natural, modified or unnatural nucleic acid oligomer that is complementary to a RNA precursor sequence.

17. A method, according to claim 12, **characterized by** that the circRNA analog is isolated from the reaction mixture by a known method of RNA isolation, such as precipitation in alcohol, size exclusion chromatography, gel electrophoresis, or high-performance liquid chromatography (HPLC).

18. Use of a circRNA analog according to claims 1-11 in protein production in an *in vitro* setup, which includes but is not limited to artificial reaction mixture, cellular lysates, nuclear lysates, cell cultures, and tissue cultures.

19. A circRNA analog according to claims 1-11 for use in therapeutic or diagnostic method, in particular for protein production in an *in vivo* setup, which includes but is not limited to whole organisms, organs, cellular and tissue transplants.

20. A pharmaceutical composition comprising of a circRNA analog according to claims 1-11.
